(19) Europäisches Patentamt
European Patent Office
Office européen des brevets

(11) EP 1 610 176 A2

(12) EUROPEAN PATENT APPLICATION

(43) Date of publication:
28.12.2005 Bulletin 2005/52

(51) Int Cl.[7]: **G03F 7/004**, G03F 7/16, C07D 207/40

(21) Application number: 05009500.9

(22) Date of filing: 29.04.2005

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: 29.04.2004 GB 0409606
07.10.2004 JP 2004295100

(71) Applicant: SEIKO EPSON CORPORATION
Shinjuku-ku, Tokyo 163-0811 (JP)

(72) Inventors:
- Fukushima, Hitoshi Seiko Epson Corporation
Suwa-shi Nagano-ken 392-8502 (JP)
- Takiguchi, Hiroshi Seiko Epson Corporation
Suwa-shi Nagano-ken 392-8502 (JP)
- Shimoda, Tatsuya Seiko Epson Corporation
Suwa-shi Nagano-ken 392-8502 (JP)
- Masuda, Takashi Seiko Epson Corporation
Suwa-shi Nagano-ken 392-8502 (JP)
- Bushby, Richard James
Leeds, LS2 9JT (GB)
- Evans, Stephan
Leeds, LS2 9JT (GB)
- Jeyadevan, J. P.
P.O. Box 57 (LK)
- Critchley, Kevin,
School of Physics and Astronomy
Leeds, LS2 9JT (GB)

(74) Representative: HOFFMANN EITLE
Patent- und Rechtsanwälte
Arabellastrasse 4
81925 München (DE)

# (54) UV decomposable molecules and a photopatternable monomolecular film formed therefrom

(57) [Problem]

The invention provides a method of controlling the solid-state property of the solid-phase surface or controlling forming reactive region. The method is attained by using a means ejecting droplets and a molecule for inclusion in a SAM which can be photo-patterned in a short period of time using low energy UV radiation, that is UV radiation having a relatively long wavelength.

[Means to Solve the Problem]

The invention provides monomolecular film that is formed from molecules comprising a structural component (B) which is hydrophobic and/or lipophobic, and a structural component (A) which decomposes when irradiated with UV light having a wavelength in the range 254-400 nm to cleave away a part of the molecule comprising the structural component (B) leaving a residual hydrophilic structural component (C). Further, the invention provides a method of forming a film pattern comprising; at least a step of ejecting a droplet, which includes a compound represented as the following Formula (0), on a solid-phase surface having a functional moiety;

[Formula 0]

**X-Y-Z (0)**

Where, X represents a structure having reactivity to a functional moiety which exists at the solid-phase surface, Y represents a decomposable structure by itself and Z represents a structure which is capable of changing solid-state properties on the solid-phase surface or a reactive structure.

EP 1 610 176 A2

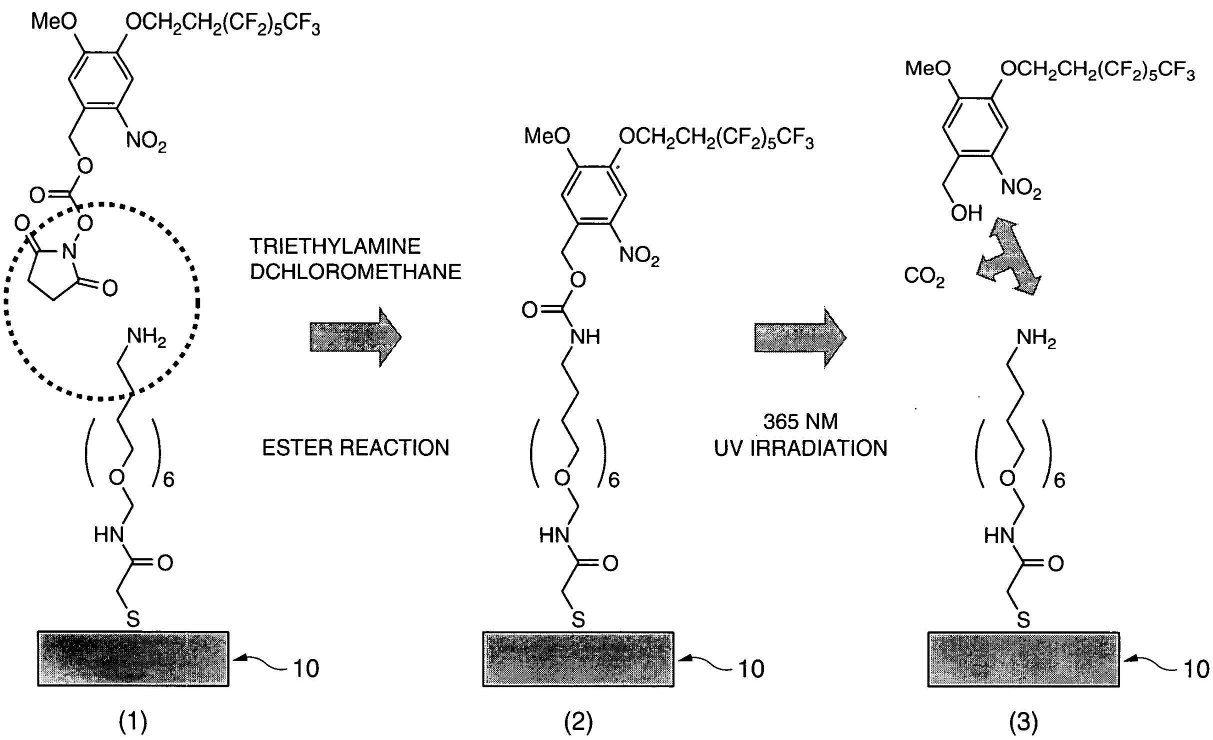
TRIETHYLAMINE
DCHLOROMETHANE
ESTER REACTION
365 NM
UV IRRADIATION
10
10
10
(1)
(2)
(3)

FIG. 2

## Description

Technical Field

The present invention relates to certain molecules which are susceptible to decomposition by UV light particularly those having a hydrophobic and/or lipophobic structural component. Such molecules may be used to form a monomolecular film which can be photo-patterned when image-wise exposed to low energy UV irradiation. Further, the present invention relates a method of forming a film pattern using a means of discarding tiny droplets such as an ink-jet method, particularly a method of forming a film pattern which is capable of changing solid-state property of the solid-phase surface, or a method of forming a film pattern having reactivity.

Background of the Invention

A number of methods are known for forming patterned films on substrates such as semiconductor substrates. One of these methods is based upon dissolving a photosensitive polymer material in a solvent and then forming a film of this material by spin coating on a semiconductor substrate. The resulting film is then irradiated with ultraviolet rays (UV) through a patterned mask resulting in the formation of a negative or a positive pattern in the photosensitive polymer film. However, the technique of spin coating is inefficient insofar as up to 99% by weight of the polymer material is discarded during the coating step.

As an improvement of such processes, it has been proposed to form photopatternable films of monomolecular thickness which can be imaged by exposure to UV irradiation. These films are called Self-Assembled Monolayers (SAMs). Examples of such methods are disclosed in Micropatteming of organosilane self-assembled monolayers using vacuum ultraviolet light at 172 nm: resolution evaluation by Kelvin-probe force microscopy by H. Sugimura *et al.,* (non patent document 1) and Scanning probe nanolithography by H. Sugimura (non document patent 2.) Methods using SAMs reduce wastage of the photopatternable material compared to processes which make use of spin coating. This is because only a very small amount of photosensitive material is needed, i.e. the amount needed to form a monomolecular film of the material.

However, the previously proposed photopatternable SAMs have the disadvantage that they have needed to be exposed to high energy UV irradiation for a long period of time in order to be satisfactorily imaged. This means that they have relatively poor processing efficiency. It is desirable therefore to provide an improved material for forming a SAM which can be photo-patterned by exposure to low energy UV irradiation, that is by exposure to UV light having a relatively long wavelength, for a short period of time.

Separately from the above, Dunkin *et al,* disclose in J. Chem. Soc. Perkin Trans. 2, (2001), page 1414 (non patent document 3) that an o-nitrobenzyl ester derivative absorbs UV radiation at approximately 254 nm. This absorption induces photoisomerization and photodecomposition reactions.
This is illustrated in the following reaction scheme:

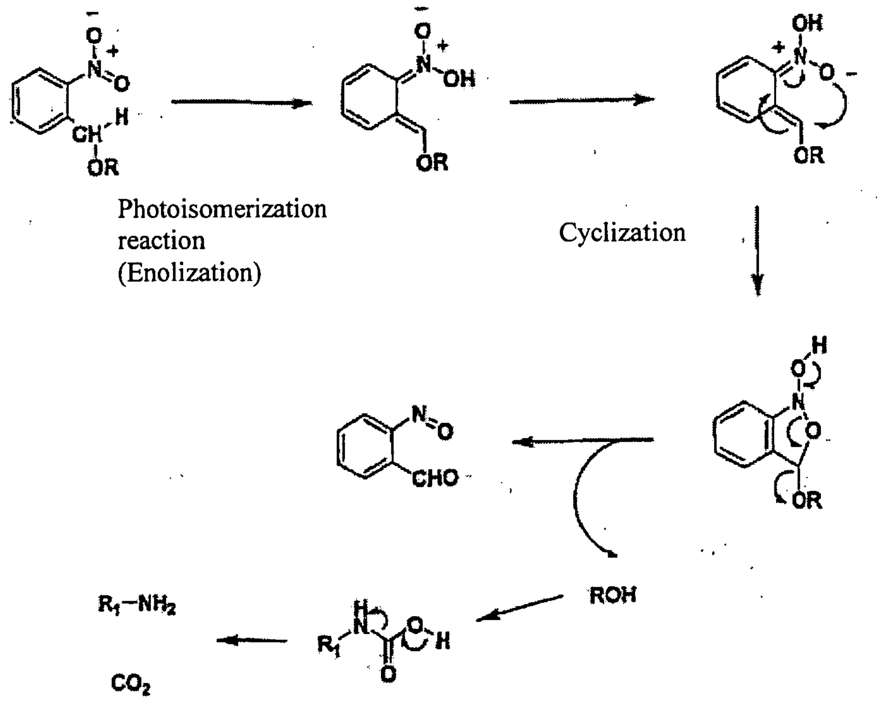


R=$R_1$NHCO-

The first reaction in the above scheme is the intramolecular enolization reaction of an o-nitrobenzyl ester derivative. This enolization reaction in turn induces intramolecular cyclization. An ester group present at the benzyl group then dissociates thereby forming a compound having an aldehyde and a nitroso group as a decomposition product. When a carbamic ester linkage is formed at the benzyl group, an amine compound and carbon dioxide are also formed as the other decomposition products.

The present inventors had the idea that derivatives of this type including a photodegradable structural component could be incorporated into a SAM useful for photo-patterning which could be formed on a variety of different substrate types. They further had the idea that such a SAM could be arranged to express two different surface properties of on the one hand hydrophobicity and/or lipophobicity and on the other hand hydrophilicity by means of careful molecular design such that when the molecule is subjected to UV irradiation, the hydrophobic and/or lipophobic structural component is cleaved away liberating a hydrophilic substituent.

In recent years, a technology is required in which DNA, a biological molecule such as antibody and compounds having reactivity with them are fixed onto a solid-phase surface with high dense and high accurate pattern so as to develop a biosensor detecting a specific biological molecule from a test sample and lab-on-chip technology implementing micro- sized biochemical experiments on a glass chip.

As a method of fixing DNA onto a substrate surface with high density, it has been proposed in the non patent document 4 that light is irradiated on a self -assembled monomolecular film having an photo- cleaved protective group with using a photo mask for example forming a pattern of a hydroxyl group , which is specifically reactive, then compounds having four different bases are reacted each other extending one DNA chain.

A method using a photo mask, however, faces difficulty in adjustment of alignment at the time of exposure reaction, resulting in low yield and high manufacturing cost with insufficient detecting sensitivity. On the other hand, as a method of forming tiny film pattern on a surface of solid substrate, a means of ejecting micro droplets such as ink-jet method is used. The means of ejecting micro droplets enables a material included in a droplet to be supplied to a specific position on the substrate by ejecting a droplet with moving a stage on which a solid phase group is installed.

Therefore, in such case when biological molecules or compounds are fixed with giving direct pattern, it is efficient that pre-processes such as forming a region being reactive with these biological molecules or compounds and controlling a solid-state property such as wettabililty, are implemented to a surface of a substrate. According to such processes, preferably trapping a material to be fixed in a targeted region and preventing it from being attached to other region can be possible.

Non Patent Document 1:
Pages 169-170 in Surface Coating Technology (2003)
Non Patent Document 2:

Page 1182 of Vol.70, in OYO BUTURI (Applied physics in Japan) (2001)
Non Patent Document 3:
Page 1414 in J. Chem. Soc., Perkin Trans.2, (2001)
Non Patent Document 4: Pages 5022 to 5026 in *Proceedings of the National Academy of Sciences.* Vol. 91 (1994) by Pease, A. et al.

Disclosure of the Invention

Problem to Be Solved

A first object of the present invention aims to provide a molecule for inclusion in a SAM which can be photo-patterned in a short period of time using low energy UV radiation, that is UV radiation having a relatively long wavelength.
A second object of the present invention aims to provide such a molecule which can be formed into various types of SAMs and used for photo-patterning a variety of different types of substrates.
A third object of the present invention aims to provide a SAM having two different surface properties, that is initially a hydrophobic and/or lipophobic property but which is converted to a hydrophilic property on UV irradiation.
A fourth object of the present invention is to provide a method for controlling a solid state property of a solid-phase surface using a means of ejecting a droplet and a method of controlling the formation of a reactive region.

Means to Solve the Problem

The present inventors found that these objects could be attained by introducing a structural component in a SAM which is decomposed when irradiated with UV light having a wavelength in the range 254-400 nm causing cleavage of the molecule whilst at the same time also including a structural component in the SAM which is hydrophobic and/or lipophobic.
Therefore, according to a first aspect, the present invention provides a molecule comprising a structural component (A), which decomposes when irradiated with UV light having a wavelength in the range 254-400 nm, and a structural component (B) which is hydrophobic and/or lipophobic.
It is preferred that the structural component (A) is an o-nitrobenzyl ester. This structural component has the advantage of being easily cleaved when irradiated with low energy UV light having a relatively long wavelength well within the range of 254-400 nm. It is preferred also that the terminal group bonded to the benzyl group of the o-nitrobenzyl ester via the ester group should be a succinic imide.
This structural component has the advantage of allowing the molecule to be coupled by means of covalent bonding either directly to a substrate surface having suitable functional groups or via a monolayer of a coupling compound previously attached to the substrate surface and which has a functional group reactive with a succinic imide residue.
The structural component (B) is preferably a fluorinated carbon atom chain which more preferably may be saturated. The chain may be straight or branched. Further the chain may also be perfluorinated which has the advantage of increasing the chain's hydrophobicity. It is preferred that the fluorinated carbon atom chain has 3 to 12 carbon atoms and 7 to 25 fluorine atoms. It is even more preferred that the fluorinated carbon atom chain has 6 to 10 carbon atoms and 13 to 21 fluorine atoms.
In this first aspect of the present invention, the molecule preferably has the general Formula (I).

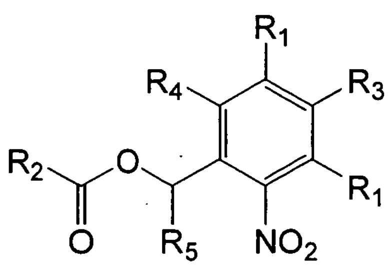

(I)

wherein:

$R_1$, independently represents a hydrogen atom, $-OR_6$ (wherein $R_6$ is an alkyl chain having a carbon number of 1 to 10, which may be a linear, branched or cyclic alkyl chain),$-NH(CO)R_7$ (wherein $R_7$ represents an alkyl chain having a carbon number of 1 to 10, which may be a linear, branched or cyclic alkyl chain, or an alkyl fluoride chain

having a carbon number of 1 to 10 and preferably 3 to 21 fluorine atoms), $N(R_8)_2$ (wherein the two substituent groups $R_8$ can be the same of different from each other and wherein each $R_8$ represents an alkyl chain having a carbon number of 1 to 5) or $-S(R_9)$ (wherein $R_9$ represents an alkyl chain having a carbon number of 1 to 10).

$R_2$ represents an N-hydroxy succinic imide-derived group (optionally substituted with a substituent containing a sulfonyl group), which is preferably linked to the ester group via the oxygen of the N-hydroxy moiety;

$R_3$ represents $-X_2-(CH_2)_n-X_1$, wherein $X_2$ represents $-CH_2-$ or -O-, n is 0 or an integer of 1 to 10, and $X_1$ represents -OZ, -Z or

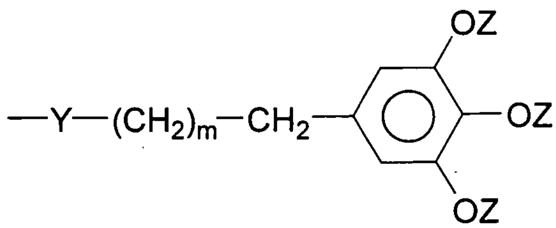

wherein:

Y represents $-(CH_2)-$ or -O-, Z represents a fluoroalkyl group having a carbon number of 1 to 20 and preferably 3 to 41 fluorine atoms, which may be linear, branched or cyclic, m represents 0 or an integer of 1 to 10; $R_4$ represents $-NO_2$ or a hydrogen atom; and $R_5$ is a hydrogen atom or an alkyl group having a carbon number of 1 to 10, which may be a linear, branched or cyclic alkyl chain.

Preferably the substituent $R_1$ is a methoxy group. The presence of the substituent $R_1$ has the advantage of causing the o-nitrobenzyl ester structural component to absorb UV light in the region 330-360 nm, that is UV light of very low energy.

Preferred fluoroalkyl groups having a carbon number of 1 to 20 represented by Z include $-(CH_2)_m(CF_2)_pF$ or a branched chain isomer thereof wherein m is as defined above and p is 0 or an integer of 1 to 9. The branched isomer may have two or more branches, and it is not necessary that each branch contains both $(CH_2)$- as well as $(CF_2)$-moieties, provided that the overall composition of the branched fluoroalkyl group is in agreement with that of the above general formula.

A preferred molecule of the above general Formula is:

[Formula 7]

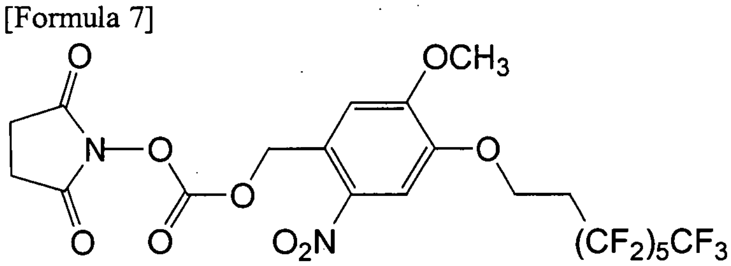

According to a second aspect, the present invention provides a monomolecular film coated substrate. The film is formed from molecules comprising a structural component (B) which is hydrophobic and/or lipophobic, and a structural component (A), which decomposes when irradiated with UV light having a wavelength in the range 254-400 nm to cleave away a part of the molecule comprising the structural component (B) leaving a residual hydrophilic structural component (C).

In the same way as in the first aspect, it is preferred that the structural component (A) is an o-nitrobenzyl ester, and that the structural component (B) comprises a fluorinated chain which may more preferably be saturated. The fluorinated chain may also be branched and/or perfluorinated.

It is preferred that the hydrophilic structural component (C) includes an amine group or a hydroxyl group. Such groups are advantageous insofar as they can react with a succinic imide to covalently bond with molecules in accordance with the first aspect of the present invention but then cleave when irradiated with UV irradiation to liberate

the relatively hydrophilic amine group or hydroxyl group.

The monomolecular film (SAM) of the second aspect is obtainable by coating a substrate with a monomolecular film of a coupling compound (D) which comprises a hydrophilic moiety and subsequently reacting the hydrophilic moiety with a molecule according to the first aspect of the present invention. The substrate may be any suitable material including a metal, a semiconductor or a plastics.

Alternatively, the monomolecular film (SAM) coating the substrate may be formed entirely from the molecules according to the first aspect of the present invention if these molecules are able to satisfactorily adhere to the substrate for instance by means of covalent bonding to suitable pre-existing functional groups on the substrate surface. This formation can in particular be the case if the substrate has a hydrophilic surface.

According to a third aspect, the present invention provides a method of photo-patterning the monomolecular film coated substrate according to the second aspect described above. The method comprises the step of image-wise irradiating the monomolecular film coated substrate with UV light having a wavelength in the range 254-400 nm through a patterned mask to cleave the coated molecules at the structural component (A) thus removing the structural component (B) from the coated film in the irradiated areas, thereby converting them from being hydrophobic and/or lipophobic to yield hydrophilic structural component (C).

Further, in order to solve the aforementioned issues attaining the above fourth object of the invention, the inventors found as the result of extensive research a compound including the structural component X, which is reactive with functional group on a solid surface, the structural component Z, which changes a solid-state property of a solid-phase surface and the structural component Y, which is decomposable and located between X and Z. The compound is applied to the solid-phase surface by a means of ejecting micro droplets containing said compound. This permits controlling the solid-state property of the solid-phase surface in line with a fine pattern. In the above compound, Z is made to be a reactive structural component forming a reactive region that may yield fine patterns on the solid-phase surface when exposed to irradiation.

Further, they found that when Y is decomposed after fixing the above compound onto the solid-phase surface, the solid-state property or reactivity of the solid-phase surface will be changed due to the exposure of a functional moiety on the surface such that it is the same or similar to the property or reactivity of the solid-phase surface prior to the reaction with the above compound.

Namely, according to the fourth aspect, the invention relates to a method [1] of forming a film pattern comprising; at least a step of ejecting a droplet, which includes a compound represented by the following Formula (0), on a solid-phase surface having a functional moiety;

$$X\text{-}Y\text{-}Z \qquad (0)$$

Here, X represents a structural component having reactivity with a functional moiety, which exists at the solid-phase surface, Y represents a decomposable structural component by itself (e.g., a structural component which can be dissociated by UV exposure under appropriate conditions) and Z represents a structural component which is capable of changing solid-state properties on the solid-phase surface or a reactive structural component. Further the invention includes the method [1] in which the solid-state property is wettability. Or the invention relates to a method [3] based on [1] or [2], wherein Z includes a structural component, which is selected from any of groups including a saturated or unsaturated alkyl chain capable of having a substituent, a saturated or unsaturated fluorinated chain capable of having a substituent, hydoxyl group, amino group, urethane group, carboxyl group, carbonyl group, urea group, sulfonic group, disulfide group, epoxy group, carbodiimide group, maleimido group, N-hydroxy succinic imide group. Or the invention relates to

a method [4] based on any of [1] to [3], wherein X includes a structural component, which is selected from any of groups including amino group, urethane group, carboxyl group, carbonyl group, urea group, sulfonic group, disulfide group, epoxy group, carbodiimide group, maleimido group, alkoxy silane, silane halide, and N-hydroxy succinic imide group. Or the invention relates to a method [5] based on any of [1] to [4], wherein Y is a structural component having a property of optical response. Or the invention relates to a method [6] based on [1], where the compound is expressed as the following Formula (I);

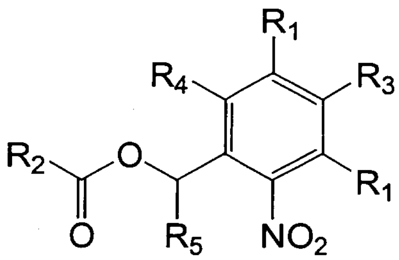

(I)

wherein:

$R_1$ independently represents a hydrogen atom, $-OR_6$ (wherein $R_6$ is an alkyl chain having a carbon number of 1 to 10, which may be linear, branched or cyclic), $-NH(CO)R_7$ (wherein $R_7$ represents an alkyl chain having a carbon number of 1 to 10, which may be linear, branched or cyclic, or an alkyl fluoride chain having a carbon number of 1 to 10, which may be linear, branched or cyclic, and which preferably has 3 to 21 fluorine atoms), $N(R_8)_2$ (wherein the individual substituents $R_8$ can be the same or different from each other, and wherein each $R_8$ represents an alkyl chain having a carbon number of 1 to 5, which may be linear, branched or cyclic) or-$S(R_9)$ (wherein $R_9$ represents an alkyl chain having a carbon number of 1 to 10).

$R_2$ represents an N-hydroxy succinic imide-derived group (optionally substituted with a substituent containing a sulfonyl group), which is preferably linked to the ester group via the oxygen atom of the N-hydroxy unit;

$R_3$ represents $-X_2-(CH_2)_n-X_1$, wherein $X_2$ represents $-CH_2-$ or -O-, n is 0 or an integer of 1 to 10, and $X_1$ represents -OZ, -Z or

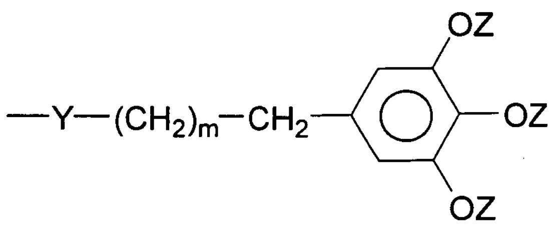

wherein:

Y represents $-(CH_2)-$ or -O-, Z represents a fluoroalkyl group having a carbon number of 1 to 20, which may be linear, branched or cyclic and preferably 3 to 41 fluorine atoms, m represents 0 or an integer of 1 to 10; $R_4$ represents $-NO_2$ or a hydrogen atom; and $R_5$ is a hydrogen atom or an alkyl group having a carbon number of 1 to 10, which may be linear, branched or cyclic.

Or the present invention relates to a method [7] based on any of the above [1] to [6], which further comprises; a step of fixing a compound having a functional moiety, which is capable of bonding X, onto the solid-phase surface before ejecting a liquid including a compound represented as the Formula. Or the present invention relates to a method [8] based on any of the above [1] to [7], wherein the method of ejecting droplets is an ink-jet method. Or the present invention relates to a method [9] based on any of the above [8] to [1], wherein the droplets include a solvent, which is selected from the group consisting of ethanol, DMF, DMSO, HMPA, pyrrolidone group, dioxane. Or the present invention relates to a method [10] based on any [1] to [9], wherein the method of ejecting droplets includes a means of controlling to dry the droplet.

[Effects of the Invention]

According to the present invention, it can provide a molecule for inclusion in a SAM, which can be photo-patterned in a short period of time using low energy UV radiation, that is UV radiation having a relatively long wavelength. Further, the present invention can provide such a molecule, which can be formed into various types of SAMs and used for photo-patterning a variety of different types of substrates. The present invention can also provide a SAM having two different surface properties, that is initially a hydrophobic and/or lipophobic property but which is converted to a

hydrophilic property on UV irradiation.

According to the present invention, a compound including a first structural component, which is reactive with functional moiety on a solid -phase surface, a second structural component, which changes a solid-state property of a solid-phase surface, and a third structural component, which is located between the first and the second structural components and decomposable, is ejected as a solution onto the solid-phase surface. It is possible to control the solid-state property of the solid -phase surface in line with minute patterns. It is also possible to control the formation of a reactive region. Further, the decomposition of the above third structural component, which is decomposable, can permit to reverse any changes of the solid-state properties and reactivity, which are due to the coating with said compound, such that the original state or a new solid-state property and a new reactivity are created at the solid-phase surface by a functional moiety, which has been exposed to the surface through the decomposition.

Brief Description of the Drawings

**[0037]** [0041 a]

Figure 1 shows the synthesis of a molecule (compound 1) according to the first aspect of the present invention

Figure 2 shows the covalent bonding of a molecule according to the first aspect of the present invention to a coupling compound (D) previously attached to a substrate and their subsequent cleavage when subjected to UV irradiation.

Figure 3 shows the UV irradiation energy distribution which can be used to expose a monomolecular film coated substrate in accordance with the second aspect of the present invention.

Figure 4 shows explanation of an optical response of a compound used in the fourth aspect of the invention.

Figure 5 is a diagram showing fixing an amino group onto the solid-phase surface.

Figure 6 is a diagram showing fixing a compound (II) onto an amino group of the solid-phase surface.

Figure 7 is diagram showing observed water -shedding property of the compound (II).

Figure 8 is diagram showing observed water -shedding property of the compound (II).

Figure 9 is diagram showing observed water -shedding property of the compound (II).

Figure 10 is diagram showing observed water -shedding property of the compound (II).

Figure 11 is a diagram showing fixing an amino protective group onto an amino group of the solid-phase surface.

Figure 12 is a diagram showing decomposing a compound (II) by irradiating UV onto the solid-phase surface.

[Description of Preferred Embodiments]

Embodiments of the invention will now be described with reference to the accompanying drawings.

Returning to the first aspect of the present invention as discussed above, this relates in its broadest aspect to a molecule comprising a structural component (A) which decomposes when irradiated with UV light having a wavelength in the range 254-400 nm, and a structural component (B) which is hydrophobic and/or lipophobic. The decomposition of the compound is preferably measured by irradiating the test compound in its crystal form, dissolved in an appropriate organic solvent or in the form of a monolayer film on a support for a period of time of from 10 s to 4000 s with UV light in the range of from 254 nm to 400 nm and an intensity of 50 $mW/cm^2$ at the absorption maximum. It is best tested by providing the compound in toluene, and irradiating it with a source of light having an intensity of 50 $mW/cm^2$ at a wavelength in the range of 254 to 400 nm for a duration of 3600 seconds. It is to be understood that the irradiation is to be effected at the absorption maximum of the compound (within the specified wavelength region). For example, the amount of decomposed compound in solution is monitored by the reduction of the specific UV absorbance (365nm) using an instrumentation of UV-visible absorption spectrum. The reduction should be at least 70%, preferably 80% from the original intensity of UV absorbance. In the case of observation of a monomolecular film, the characterization takes place using Fourier Transformation infrared spectrum (FTIR) spectrometer equipped with refraction absorption spectrum system. The actual photodecomposition is monitored by the reduction of the specific IR intensity ($1525cm^{-1}$; exhibited by the vibration of the $-NO_2$ group) on the surface of the UV-irradiated monomolecular film. The reduction should be 70%, preferably 80% from the original intensity of the IR intensity. Thus the molecule comprises at least two separate structural components. The first of these structural components, labeled (A), is a chemical structure or moiety which decomposes when irradiated with UV light having a relatively long wavelength which falls in the range 254-400 nm. This structural component renders the molecule sensitive to UV light and thus makes the molecule itself, or a larger molecule that is sensitive to UV light and therefore capable of being photo-patterned. The structural component (A) is coupled into the larger molecule. On the other hand, the structural component (B) is hydrophobic and/or lipophobic resulting in the molecule itself having this property or these properties. As a consequence, a monomolecular film formed from the molecules of the first aspect of the present invention has a hydrophobic and/or lipophobic surface property. The property is also possessed by a monomolecular film (SAM), which is formed by coupling the molecules of the first aspect of the present invention to a monomolecular film of a coupling compound already coated on a substrate.

Molecules in accordance with the first aspect of the present invention can be photo-patterned even when irradiated for a short period of time such as 10 seconds to 10 minutes, more preferably 30 seconds to 5 minutes, most preferably 1 to 5 minutes with low energy UV light corresponding to a wavelength in the range 254-400 nm. This means that photo-patterning can be carried out relatively efficiently insofar as it requires only relatively low energy irradiation.

The structural component (A) which can be decomposed when irradiated with UV light may be an o-nitrobenzyl ester. The decomposition of such a structural component is set out above in relation to the acknowledged prior art of Dunkin *et al.*

The structural component (B) is hydrophobic and/or lipophobic. The presence of this structural component causes a monomolecular film formed from the molecules to be in turn hydrophobic and/or lipophobic. Suitable examples of the structural component (B) are long chain hydrocarbons and long chain fluorinated carbon chains which may be perfluorinated or substituted by a mixture of fluorine and hydrogen atoms. Preferably such chains are saturated and/or branched, that is they may have a dendritic structure. Preferably, the optionally fluorinated carbon chains comprise 1 to 12, more preferably 3 to 10 carbon atoms.

Examples of such chains include the saturated fluorinated chains $-(CH_2)_n(CF_2)_mCF_3$,$-(CH_2)_nCF[(CF_2)_mCF_3]_2$ and $-(CH_2)_nC[(CF_2)_mCF_3]_3$, (where n and m are integers, preferably 0 to 10 and 0 to 9, respectively.)

If the structural component (A) is an o-nitrobenzyl ester, then the structural component (B) can be easily introduced at the para-position of the benzyl group via an ether bond.

If the structural component (A) is an o-nitrobenzyl ester, then the molecule of the first aspect of the present invention preferably has a succinic imide structure as the terminal group of the benzyl group due to its advantageous reactivity. This succinic imide structure enables the resulting molecule to be easily coupled to a substrate which either has suitable surface reactive groups such as hydroxyl or amino groups, or may be coupled to such a substrate by means of a monolayer of a coupling compound (D) previously coated and/or bonded to the substrate which has a functional group reactive with the succinic imide structure such as a hydroxyl or amino group. This enables the molecules of the first aspect of the present invention to be applied to practically any type of substrate such as the surface of a gold substrate which is surface-modified with amino groups, a surface of a semiconductor substrate such as silicon, an organic material surface of a plastics substrate or the surface of an insulating substrate. In this way, a photodegradable SAM can be produced.

For instance, a substrate such as a gold film may be coated with a monolayer of an aminosilane compound such as 3-aminopropyltrimethoxysilane which is then coupled to a molecule according to the first aspect of the present invention including an o-nitrobenzyl ester having a terminal succinic imide residue. Such a coupling mechanism enables the molecules of the first aspect of the present invention to be widely applied to many different types of substrate and so used in photo-patterning applications. The coupling compound (D) could have an alternative substituent reactive with a succinic imide residue such as a hydroxyl group.

This coupling concept is more fully illustrated in FIGS 2(1) and 2(2). In these Figures, a substrate (10) is initially treated with $HSCH_2CONHCH_2(OCH_2CH_2)_6CH_2CH_2NH_2$ as a coupling compound (D) which forms a monomolecular film (not illustrated) on the substrate (10). The film's surface includes reactive amino groups. These amino groups may then be reacted with molecules according to the first aspect of the present invention which bear a succinic imide residue at one terminal which reacts together with the amine substituent of the coupling compound (D) to form a carbamic ester structure. The reaction between the coupling compound and the molecule according to the first aspect of the present invention can be performed for example in an organic solvent such as dichloromethane using trimethylamine as a reaction catalyst. The substrate (10) may for instance be a metal substrate such as gold or silver since such metal substrates have relatively strong reactivity with thiols and disulphides. Therefore the coupling compounds readily react with the substrate to form a SAM on the substrate surface. An alternative coupling compound would be 11-aminododecanethiol. Preferably, the coupling compound (D) is selected such that it contains a first terminal bonding group, which is capable of reacting with the molecule of the present invention. Moreover, compound (D) preferably

contains a linker group as well as a second terminal bonding group. The linker group can be any divalent group having preferably a length equivalent to that of a linear alkyl group of 2 to 18 carbon atoms, and a preferred embodiment is the amino ethane thiol linker group. The second terminal bonding group is preferably capable of bonding to the surface of the support. This can either be accomplished by forming a covalent bond with functional groups present at the surface of the support, or the bonding can take place by means of other interactions such as electrostatic interactions. Accordingly, the structure of the second terminal bonding group is preferably determined by the structure and properties of the surface of the support, to which compound (D) is to be attached. Typical examples of support surfaces and suitable second bonding groups are as follows: The amino moiety (-NH2) is preferred as the first core surface group. The amino ethane thiol is a practically suitable molecule to fabricate the support surface on an Au substrate. In use of other metal surfaces such as oxidized Si, (3-aminopropyl)triethoxysilane is highly preferred as chemical substance to prepare an ideal reactive amino group on substrate surfaces. The suitable second bonding groups are chosen in line with the primary amino group of the core support surface. The N-hydroxy succinimide ester moiety is highly reactive with primary amino groups to proceed with the formation of amide ester linkages between the core surface and the multi functional unit I, which consists of structural component (A) and (B). This N-hydroxy succinimide ester group is introduced into the tail end of molecular unit I.

The molecule of the first aspect of the present invention preferably has the general Formula (I).

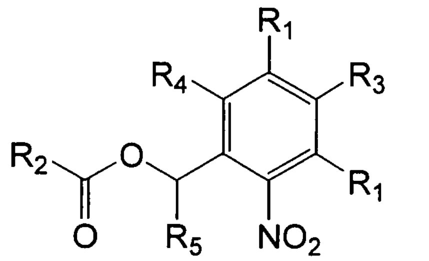

(I)

wherein:

$R_1$ independently represents a hydrogen atom, $-OR_6$ (wherein $R_6$ is an alkyl chain having a carbon number of 1 to 10, which may be linear, branched or cyclic), $-NH(CO)R_7$ (wherein $R_7$ represents an alkyl chain having a carbon number of 1 to 10, which may be linear, branched or cyclic, or an alkyl fluoride chain having a carbon number of 1 to 10, which may be linear, branched or cyclic and which preferably has 3 to 21 fluorine atoms), $N(R_8)_2$ (wherein the two substituents $R_8$ can be the same or different from each other and wherein each $R_8$ represents an alkyl chain having a carbon number of 1 to 5, which may be linear, branched or cyclic, or wherein the two substituents $R_8$ can be linked to form a 3 to 11-membered cyclic structure together with the nitrogen atom) or $-S(R_9)$ (wherein $R_9$ represents an alkyl chain having a carbon number of 1 to 10).

$R_2$ represents an N-hydroxy succinic imide-derived group (optionally substituted with a substituent containing a sulfonyl group) wherein the group is bonded to the ester group via the oxygen atom of the N-hydroxy moiety;

$R_3$ represents $-X_2-(CH_2)_n-X_1$, wherein $X_2$ represents $-CH_2-$ or -O-, n is 0 or an integer of 1 to 10, and $X_1$ represents -OZ, -Z or

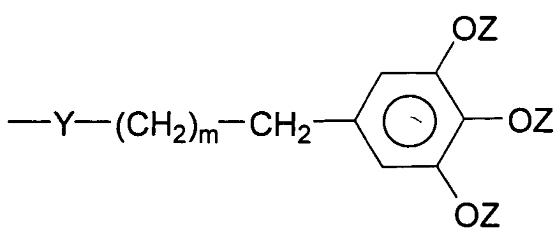

wherein:

Y represents $-(CH_2)-$ or -O-, Z represents a fluoroalkyl group having a carbon number of 1 to 20, which may be linear, branched or cyclic, and which preferably has 3 to 25 fluorine atoms, m represents 0 or an integer of 1 to 10; $R_4$ represents $-NO_2$ or a hydrogen atom; and $R_5$ is a hydrogen atom or an alkyl group having a carbon number

of 1 to 10, which may be linear, branched or cyclic.

Such compounds are efficiently decomposed even when absorbing a small amount of UV radiation but enable a SAM to be formed having a high surface hydrophobic and/or lipophobic property.

It is particularly preferred in the above compounds of Formula (I) that the substituent $R_1$ should be a methoxy group ($-OCH_3$). This substitent is a structure in which a methoxy group is bonded to the para-position with respect to the nitro group. This substitution causes the compound to strongly absorb UV radiation in the wavelength range 330-360 nm. It is further preferred that $R_5$ is a hydrogen atom.

It is further preferred in the above compounds of general Formula (I) that the substituent Z is $-(CH_2)_m(CF_2)_PF$ or a branched chain isomer thereof wherein m is as defined above and p is 0 or an integer of 1 to 9.

If the molecules according to the first aspect of the present invention are coated as a monolayer (a SAM) on a substrate having a hydrophilic surface, then the resulting monomolecular film coated substrate may be directly subjected to photo-patterning by exposure to UV irradiation. Thus UV irradiation will cleave the molecule at the structural component (A) exposing, after rinsing, the hydrophilic surface of the substrate. Areas of the monomolecular film which are masked from irradiation remain hydrophobic and/or lipophobic due to the structural component (B). As a consequence, such a coated substrate is able to demonstrate two different surface properties. Namely, there is a hydrophilic surface property in areas which have been subjected to UV irradiation and a hydrophobic and/or lipophobic surface property in other areas where they have not.

Alternatively, the molecules according to the first aspect of the present invention may be coupled to the surface of a substrate through a coupling compound (D) such as illustrated in FIG. 2(2). If the molecule of the invention contains an N-hydroxy succinimide-derived structure, the resulting SAM has a carbamic ester structure. When the SAM surface is irradiated with UV radiation having a wavelength of for instance 365 nm through a photo-mask, photodecomposition of the SAM proceeds quite quickly, in about 30 seconds to 5 minutes, and the carbamic ester structure is decomposed. Subsequently, the film surface is rinsed with a suitable solvent such as water, and the original amino groups of the coupling compound (D) reappear on the film surface as illustrated in FIG. 2(3). As a consequence, a large difference exists in the surface energy between the UV irradiated regions of the SAM coated substrate and the non-irradiated regions.

In accordance with the above, a monomolecular film or SAM, is obtained whose wettability to a solution and solvent can be significantly altered by subjecting it to image-wise UV irradiation. For instance, when the above-described molecules containing a succinic imide residue are used, these molecules may be reacted with a hydroxy thiol, such as 11-hydroxydodecanethiol, by refluxing in acetonitrile as a solvent. This reaction produces a disulphide carbamate. Subsequently, this derivative may be fixed to a gold substrate to form a monomolecular film thereon. This coated substrate may then be irradiated with UV light having a wavelength in the range 254-400 nm in order to bring about a dissociation reaction. The reaction results in the cleavage away of the succinic imide derivative leaving only the 11-hydroxydodecanethiol, which presents hydroxyl groups on its film surface rendering the irradiated areas of the SAM hydrophilic. On the other hand, the unirradiated areas of the SAM remains hydrophobic and/or lipophobic due to the presence of the structural component (B).

As previously described, the second aspect of the present invention provides a monomolecular film coated substrate. The film is formed from molecules comprising a structural component (B), which is hydrophobic and/or lipophobic, and a structural component (A), which decomposes when irradiated with UV light having a wavelength in the range 254-400 nm to cleave away a part of the molecule comprising the structural component (B) leaving a residual hydrophilic structural component (C).

The monomolecular film (SAM), which is deposited on the substrate will be understood to be a highly functional layer having two possible surface properties that is an initial hydrophobic and/or lipophobic property, and a hydrophilic property after being subjected to UV irradiation. These properties make such a monomolecular film coated substrate suitable for photo-patterning by UV irradiation. The structural component (A) and the structural component (B) are the same as described in respect of the first aspect of the present invention above. Accordingly, unless the monomolecular film of the second aspect of the present invention is particularly described in the following, the above description of the molecules of the first aspect of the present invention can be applied.

When turning to the residual hydrophilic structural component (C), the component is a hydrophilic substituent group such as an amine group or a hydroxyl group. This group is liberated upon cleavage of the structural component (A) caused by UV irradiation. This cleavage is particularly illustrated in FIGS 2(2) and 2(3). According to this Figure, UV irradiation of the monomolecular film coated substrate of FIG. 2(2) causes cleavage of the o-nitrobenzyl ester structure. After rinsing away of the residues, the amine-substituted coupling compound (D) remains resulting in areas of the monomolecular film, which have been irradiated being hydrophilic. In contrast, areas masked from the UV irradiation will remain hydrophobic due to the chain $-OCH_2CH_2(CF_2)_5CF_3$ (or the like) which constitutes the structural component (B).

The monomolecular film in the second aspect of the present invention is preferably obtainable by coating a

substrate with a coupling compound (D), which comprises a hydrophilic moiety and subsequently reacting the hydrophilic moiety with a molecule according to the first aspect of the present invention. Alternatively, when the surface of the substrate is already hydrophilic, the monomolecular film coated substrate according to the second aspect of the present invention may be obtained simply by coating onto the substrate molecules according to the first aspect of the present invention to form a SAM. The coating of the molecule according to the first aspect of the invention and/or the coupling compound (D) can be effected by immersing the surface to be coated into a solution of the compound to be coated. The coupling of the molecule according to the first aspect of the invention to the coupling compound (D) is preferably effected by subjecting the coated surface to conditions, which initiate a chemical reaction leading to the formation of a covalent bond between the two compounds. Typical conditions are as follows: In case of a gold substrate, 1 mM of amino ethane thiol in absolute ethanol is prepared and gold substrate is immersed into the ethanol solution for 5h at room temperature The immersed gold substrate is thoroughly rinsed with absolute ethanol and dried by $N_2$ blow. Subsequently, the gold substrate is placed into the solution of the reaction unit I, which is dissolved in DMSO (dimethyl sulfoxide) and left for 2h at room temperature. Finally, the surface of the gold substrate can generate the monomolecular film including the multi functional molecular unit I.

Once the monomolecular film coated substrate has been subjected to photo-patterning, then for instance a polymer solution containing a functional material may be applied to its surface by conventional techniques such as spin coating or ink jet printing which selectively applies the solution. The functional material will be deposited over the patterned monomolecular film which in turn allows the functional material to be easily patterned.

As described above, the present invention provides in a third aspect a method of photo-patterning a monomolecular film coated substrate as described above. The method comprises the step of image-wise irradiating the monomolecular film coated substrate with UV light having a wavelength in the range 254-400 nm through a patterned mask to cleave the coated molecules at the structural component (A) thus removing the structural component (B) from the coated film in the irradiated areas converting them from being hydrophobic and/or lipophobic to hydrophilic.

Techniques of photo-patterning using light irradiation and masks are well known to those working in the field of resist technology. Reference is made to "Resolution Enhancement Techniques in Optical Lithography", F.H. Schellenberg (Editor) 2004; Society of Photo-Optical Instrumentation Engineering.

It will be appreciated that the method according to the third aspect of the present invention allows photo-patterning to take place requiring only a monomolecular film coating on a substrate. The method therefore solves the problem of substantial wastage referred to in the introduction of this specification when using conventional photosensitive polymer materials applied by spin coating. In addition, the two-stage method of forming a monomolecular film provided by the present invention which includes designing and synthesizing molecules in accordance with the first aspect of the present invention having both a UV degradable structure and a hydrophobic and/or lipophobic structural component and then coupling these two structures to a coupling compound (D) via a hydrophilic moiety on the former. The two-stage method provides a widely applicable technique for photo-patterning a variety of substrate types.

The compound (the compound (0)) of the fourth aspect of the invention is presented as following Formula (0).

$$X\text{-}Y\text{-}Z \qquad (0)$$

The end of it is provided with the structure X having reactivity with functional group on the solid-phase surface. For example, when a glass substrate is used as the solid-phase substrate, the surface of the substrate has plenty of hydroxyl groups. X can be, for example, alkoxy silane or silane halide $-SiL_n$ (where L represents alkoxy group or halide and n represents an integer number of 1 to 4.) L part is decomposed by water thereby and coupled to hydroxyl group resulting in the compound (0) being fixed onto the solid-phase surface.

X is appropriately selected to match the solid-phase materials and compounds fixed onto the surface except the above alkoxy silane or silane halide. X is not specifically limited so long as having reactivity with functional group with a solid-phase surface. But, it is preferably selected from high reactive structures including any of groups including amino group, urethane group, carboxyl group, carbonyl group, urea group, sulfonic group, disulfide group, epoxy group, carbodiimide group, maleimido group, alkoxy silane, silane halide, and N-hydroxy succinic imide group.

According to the fourth aspect of the invention, droplets including the above compound (0) are ejected onto the solid-phase surface having a functional group. Here, the functional group exposed by the solid surface may be a functional group originating from the solid phase material itself or a functional group of a compound which is fixed onto the solid surface in advance. When the solid phase surface is a glass substrate described above, a hydroxyl group of the glass substrate can be used as a functional group of the solid surface. Further, when the solid -phase material has not a functional group to be coupled with X, a compound having a functional group to be coupled with X is fixed onto the solid-phase surface in advance prior to ejecting droplets including the compound (0).

The functional group exposed by the solid -phase surface (either as a part of the material of the solid-phase itself, or as a result of attaching a compound having a functional group) is preferably selected from any of groups

including amino group, urethane group, carboxyl group, carbonyl group, urea group, sulfonic group, thiol group, sulfide group, disulfide group, epoxy group, carbodiimide group, maleimido group, alkoxy silane, silane halide, and N-hydroxy succinic imide (NHS) group.

A method of fixing a compound having these functional groups is not specifically limited. But, the end opposite to these functional groups, for example, can be coupled with a functional group for forming SAM corresponding to a solid-phase material such as the above -SiLn, for example, (where L represents alkoxy group or halide and n represents an integer number of 1 to 4.) thiol group, sulfide group, disulfide group, and others. SAM can be formed thereby and contacting a solution including the compound with the solid-phase surface and fixed.

The compound (0) is provided with the structure Z, which can change solid-state property of the solid-phase surface or reactive property at the end opposite to X.

The solid-state property of the solid phase surface can be, for instance, response to wettability, heat and pressure, configuration, viscosity, adhesion, water absorption, elasticity and others. Particularly, a structure having wettability is preferable for Z. Controlling wettabliltiy such as hydrophobicity, hydrophilicity, lipophobicity and lipophilicity enables materials to be fixed to appropriately be absorbed onto a region desired for fixing of the solid phase surface. On the other hand, fixing them onto other regions can be prevented. The structures controlling favorable wettability as Z are, for example, the following; as the structure giving hydrophobicity to the solid-phase surface, a saturated or unsaturated alkyl chain, which may be linear, branched or cyclic, preferably having 1 to 18 carbon atoms, optionally having a substituent, and a saturated or unsaturated fluorinated chain, which may be linear, branched or cyclic, preferably having 1 to 12 carbon atoms and 3 to 25 fluorine atoms, optionally having a substituent are cited. As the structure giving hydrophilicity to the solid-phase surface, hydoxyl group and amino group are cited.

Meanwhile, the favorite reactive structure as Z is a functional group having high reactivity. The group is, for example, selected from hydoxyl group, amino group, urethane group, carboxyl group, carbonyl group, urea group, sulfonic group, disulfide group, epoxy group, carbodiimide group, maleimido group, N-hydroxy succinic imide group. Further, X-Y-Z can be diluted into appropriate solvents, which are specifically not limited so long as ejected as droplets. Alternatively, reactive structure Z may also be derived from entire molecules such as avidin, biotin, antigen, antibody and protein A having biological affinity.

The compound (0) used in the fourth aspect of the present invention contains the structure Y, which is located between X and Z, and which is decomposable by itself. The decomposability of Y allows Z to be cleaved away from the solid-phase surface by decomposing Y after fixing the compound (0). This process enables solid-state property and reactive property to be restored to the original states. Further, the decomposition of Y enables the solid-phase surface to receive new solid-state property and reactivity by newly exposed atoms. A method of decomposition is not specifically limited. But, this can be attained as followings. A solution including a compound having property, which decomposes Y, is contacted with the solid-phase surface. This basic decomposition mechanism heavily depends on the photo-dissociation system of e.g., the o-nitrobenzyl moiety exposed by 365 nm of UV. Or a solution including such compound is applied to the solid-phase surface by the micro droplet ejecting means.

Y is preferably a structure having optical response. A preferred structural unit Y is shown below:

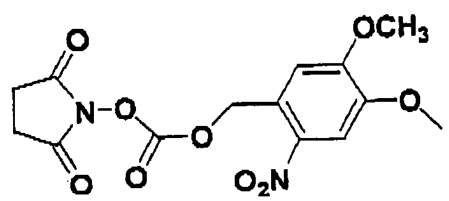

Irradiating light onto the solid phase surface easily decomposes Y thereby. Further, when using an appropriate mask, Y can be decomposed only in a part of area where the compound (0) is fixed.

The following Formula (I) is cited as a preferable embodiment for the above compound (0).

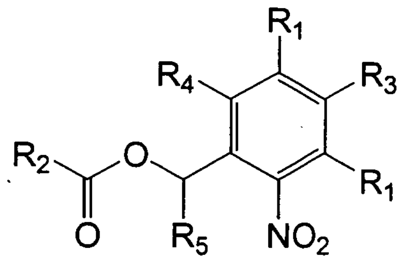

(I)

wherein:

$R_1$ independently represents a hydrogen atom, $-OR_6$ (wherein $R_6$ is an alkyl chain having a carbon number of 1 to 10, which may be linear, branched or cyclic), $-NH(CO)R_7$ (wherein $R_7$ represents an alkyl chain having a carbon number of 1 to 10, which may be linear, branched or cyclic, or an alkyl fluoride chain having a carbon number of 1 to 10, which may be linear, branched or cyclic, and which preferably has 3 to 21 fluorine atoms), $N(R_8)_2$ (wherein the substituents $R_8$ can be the same or different from each other, and wherein each $R_8$ represents an alkyl chain having a carbon number of 1 to 5, which may be linear, branched or cyclic) or $-S(R_9)$ (wherein $R_9$ represents an alkyl chain having a carbon number of 1 to 10, which may be linear, branched or cyclic).

$R_2$ represents an N-hydroxy succinic imide-derived group (optionally substituted with a sulfonyl group) bonded to the ester via the oxygen atom of the N-hydroxy group;

$R_3$ represents $-X_2-(CH_2)_n-X_1$, wherein $X_2$ represents $-CH_2-$ or -O-, n is 0 or an integer of 1 to 10, and $X_1$ represents -OZ, -Z or

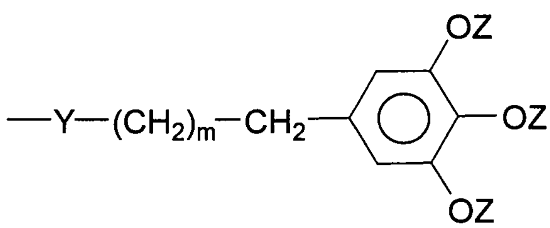

wherein:

Y represents $-(CH_2)-$ or -O-, Z represents a fluoroalkyl group having a carbon number of 1 to 20, which may be linear, branched or cyclic and which preferably has 3 to 25 fluorine atoms, m represents 0 or an integer of 1 to 10; $R_4$ represents $-NO_2$ or a hydrogen atom; and $R_5$ is a hydrogen atom or an alkyl group having a carbon number of 1 to 10, which may be linear, branched or cyclic.

In this compound, $R_2$ which is a NHS residual group, or a NHS residual group having a sulfonyl group, functions as the structure X and the compound (I) is easily fixed onto the solid- phase surface if an amino group exists as a functional group on the solid-phase surface. $R_3$ introduces fluoroalkyl chain functions as the structure Z and changes the solid-state property of the solid-phase surface to hydrophobic and/or liophobic.

Further, the compound is decomposed by absorbing ultraviolet rays of long wavelengths such as 254 nm to 400 nm, preferably by the reaction shown in FIG.4. Therefore, the compound (I) is fixed onto the solid- phase surface (10) once giving hydrophobic and/or liophobic property to the solid phase surface. Then, irradiating ultraviolet rays removes the structure Z including the fluoroalkyl chain from the solid-phase surface changing the solid-state property of the surface to be hydrophilic again.

The compounds represented by the following Formula (II) and (III) are preferred embodiments of the compound represented by the above Formula (I).

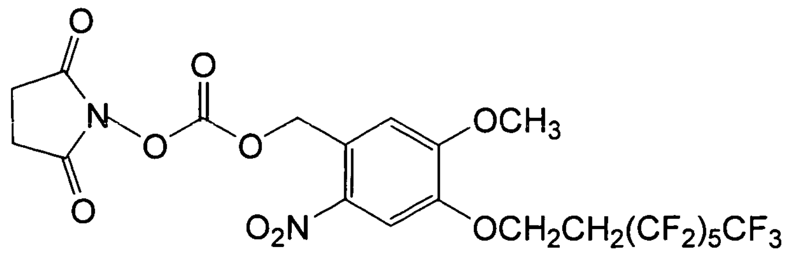

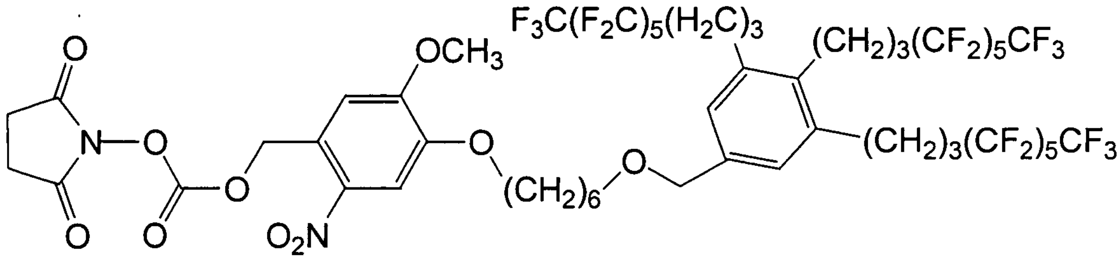

A micropipette, a micro dispenser and an ink jet method are cited as a method ejecting droplets used in the fourth aspect of the present invention. Particularly, an inkjet method is preferable for precise patterning. The ink jetting employs various ways for ejecting liquid, including piezo jet using piezo elements, thermal jet using thermal elements, and electrostatic actuation utilizing electrostatic forces between an oscillating plate and an electrode. Preferably, the piezo jet and electrostatic actuation, which have no effect of high temperature on ejected liquid, may be used with temperature-sensitive biological substances.

The solvent for the compound (0) and especially for the compound (I) used in the fourth aspect of the invention is not specifically limited if it is a liquid, which is capable of being ejected by a micro droplet ejecting means. But, it is preferably a solvent or a compound, which has high solubility to the compound (0) or (I) and low saturated vapor pressure, namely low boiling point and which is difficult to dry. As the solvent, for example, DMF, DMSO, HNPA, a pyrrolidone group solvent, and a proton polar solvent such as dioxane are cited.

Further, in the method of the fourth aspect of the invention, a means for controlling drying droplets is preferably applied when ejecting droplets. It is easy to dry droplets ejected by a micro droplet ejecting means because of a small amount of volume. Hence, in order to assure sufficient time for reacting the structure X with a functional group on the solid-phase surface, it is preferable to extend time for drying droplets. As the means for controlling drying droplets, a means for shortening a distance between droplets, a means for ejecting droplets (a solvent or solution) again prior to drying droplets, a means for adding a coating material and a means for delaying moving speed of a stage where a solid-phase is installed in case of an inkjet method are cited as examples.

**[0081]** [0089a] The preferred compounds of the present invention can be synthesized as follows. In addition, some typical experimental protocols are also provided below:

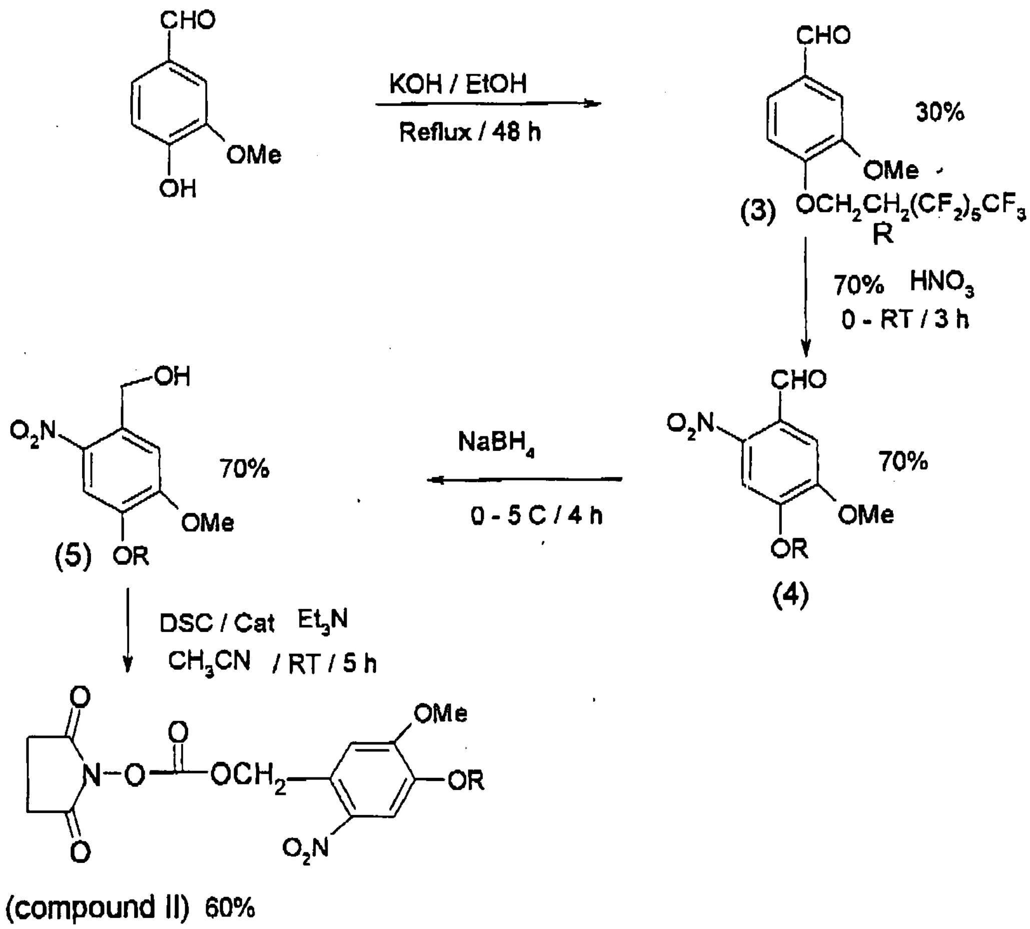


**4-(1H,1H,2H,2H-perfluorooctyloxy)-5-methoxybenzaldehyde (3)**

To a mixture of KOH (0.73 g, 13 mmol) and ethanol (10 ml) under nitrogen was added vanillin (1.0 g, 6.57 mmol). 1-lodo-1H,1H,2H,2H-perfluorooctane (5.0 g, 10.54 mmol) at 70 °C was added, and reflux for 48 h. The mixture was allowed to reach RT. The mixture was filtered and the solids were washed with $Et_2O$. Solvent was removed in vaccuo and the residue was dissolved in water. The aqueous mixture was extracted with $Et_2O$. The organic layer was washed with water and brine, dried over $MgSO_4$ and filtered, and the solvent was removed in vaccuo. Purification by flash chromatography with $^n$hexane / EtOAc (70 / 30) gave the product as white solid (0.90 g, 30%). $^1$H NMR (300 MHz, $CDCl_3$) δ: 9.87 (s, 1H, Ar-H), 7.48-7.43 (m, 2H, Ar.H), 6.99 (d, J=8.2 Hz, 1H, Ar-H), 4.43-4.38 (t J=7.14 Hz, 2H), 3.93 (s, 3H), 2.81-2.69 (m, 2H); $^{13}$C NMR (75 MHz, $CDCl_3$) 191.28, 153.26, 1.50.35, 131.28. 126.90, 112.34, 110.02, 61,54, 56.47, 31.82, 31.54, 31.25; MS (ES+) 499 ($[M+H]^+$, 55), 454 (20), 391 9220, 279 (5), 241 (5).

**2-Nitro-4-(1H,1H,2H,2H-perfluorooctyloxy)-5-methoxybenzaldehyde (4)**

Nitric acid (70%, 10 ml) was cooled to 0 °C, 4-(1H,1H,2H,2H-perfluorooetyloxy)-3-methoxybenzaldehyde, 3 (0.3 g, 0.6 mmol) was added with stirring, and the mixture was brought to RT over 3 b and then poured into ice-water (50 ml), The resultant yellow solid was collected by filtration, washed with cold water and cold ethanol, dried, and recrystallized from 95% ethanol, affording 4 (0.25 g, 80%) in the form of yellow solids. $^1$H NMR (300 MHz, $CDCl_3$) δ: 7.64 (s, 1H, Ar-H), 7.43 (s, 1H, Ar-H, 4.45 (t. J=6.77 Hz, 2H), 4.01 (s, 3H), 2.86-2.69 (m, 2H); $^{13}$C NMR (75 MHz, $CDCl_3$) 188.08, 154,01, 151.17, 126.77, 110.71, 108.91, 91.23, 62.28, 57.17. 31.78, 31.48, 31.21; MS (ES+) 544 ($[M+H]^+$, 100), 514 (15), 410 (15), 282 (10), 178 (10); Anal. calcd. For $C_{16}H_{10}F_{13}O_5N$: C 35,35%, H 1.85%, N 2.57% found C 35.35%, H1.95%, N 2.75%.

**2-Nitro-4-(1H,1H,2H,2H-perfluorooctyloxy)-5-methoxybenzylalchnl (5)**

To an ice-cooled solution of 2-Nitro-4-(1H,1H,2H,2H-perfluorooctyloxy)-5-methoxybenzaldehyde, 4 (0.3 g, 0.55 mmol) in anhydrous THF (5 ml) $NaBH_4$ (42 mg, 1.1 mmol) was added and the mixture was stirred at 0-5 °C for 4 h. The reaction was quenched by addition of water (10 ml). The organic layer was separated and the aqueous layer was extracted with EtOAc (3 x 10 ml), and the combined organics were dried over anhydrous $Na_2SO_4$. After filtration, the solvent removed under reduced pressure to give a solid which was chromatographed on a silica gel column using $^n$hexane / EtOAc (60 / 40). (0.21 g, 70%) $^1$H NMR (300 MHz. $CDCl_3$) δ: 7.74 (s, 1H, Ar-H), 7.22 (s, 1H, Ar-H), 4.98 (s, 2H), 4.37 (t. J=6.87 Hz, 2H), 3.99 (s, 3H), 1.82-2.65 (m, 2H), 2.62 (br.s, 1H); $^{13}$C NMR (75 MHz, $CDCl_3$) 154.80, 146.79, 139.89, 133.75, 111.82. 110.56, 63.18, 56.90, 31.80, 31.54, 31.26; MS (ES+) 528 ($[M-OH]^+$, 100)

**Compound II**

To a stirred solution of 2-Nitro-4-(1H,1H,2H,2H-perfluorooctyloxy)-5-methoxybenzylalcohol. 5 (0.2 g, 0.36 mmol) in anhydrous $CH_3CN$ (5 ml), under argon atmosphere, was added *N,N*-disuccinimidyl carbonate (0.1 g, 0.39 mmol) followed by $Et_3N$ (0.05 ml, 0.35 mmol). The solution was stirred for an additional 5 h and then the solvent was removed in vacuo. The residue was purified by silica gel column using DCM / EtOAc (96/4), gave the compound as pale yellow solid. (0.15 g, 62%, mp 46-48 °C). $^1$H NMR (300 MHz, $CDCl_3$) δ: 7.80 (s, 1H, Ar-H), 7.23 (s, 1H, Ar-H), 5.99 (s, 2H), 4.39 (t, J=6.73 Hz, 2H), 4.05 (s, 3H), 2.87 (s, 4H), 2.80-2.68 (m, 2H); $^{13}$C NMR (75 MHz, $CDCl_3$) 168.88,155.07,151.81, 147.36,139.23, 126.89, 110.54, 109.49, 69.48. 62.09, 57.08, 31.76. 31.51., 31.23, 25.86; MS (FAB) 686 ($[M]^+$, 8), 528 (100); HRMS (ES+) Calcd. For $C_{21}H_{15}N_2O_9F_{13}Na$ 709.0468, found 709.0473; Anal. calcd. For $C_{21}H_{15}N_2O_9F_{13}$: C :36.73%, H 2.20%, N 4.08% found C 36.45%, H 2.35%, N 4.25%.

Synthetic procedure of Compound III is as below.

First we need to synthesis $CF_3(CF_2)_5(CH_2)_3I$ for making compound III (no semifluorinated chains of this type are available commercially). With commercially available chains the alkylation of 3,4,5-trihydroxybenzaldehyde do not work.

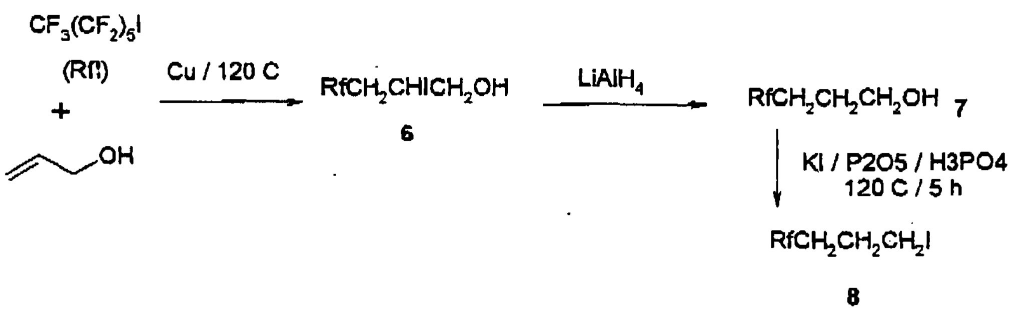


**2-Iodo-1-perfluorohexyl-3-propanol (6)[2]**

A 10 ml glass ampoule was charged with perfluorohexyl iodide (4.5 g, 10 mmol), 2-propen-1-ol (1.4 ml, 10 mmol) and copper powder (63 mg, 1. mmol). The reaction mixture was stirred at 120°C for 3 h and then dissolved in 10 ml of diethyl ether, the catalysy filtered off, the solvent evaporated. The pale yellow solid obtained was recrystallized in hexane (3.78 g, 75%). $^1$H NMR (500 MHz. $CDCl_3$) δ: 4.44 (t, 1H), 3.86-3.76 (m, 2H), 3.07-2.95 (m, 1H), 2.83-2.71 (m, 1H), 2.03 (t, 1H, OH); $^{13}$C NMR (125 MHz, $CDCl_3$) 69.64, 39.39, 39.22, 39.05. 23,26; MS (ES+) 527 ($[M+Na]^+$, 30), 406 (75). 338 (85). 193 (100); Calcd. For $C_9F_{13}H_6IO$ C 21.43%, H 1.20%, 125.18 found C 21.70%, H 1.4%, I 25.35%.

**3-perfluorohexyl-1-propanol (7)**

To a slurry of $LiAlH_4$ (0.8 g, 21 mmol) in 10 ml $Et_2O$ was added slowly 6 (5 g, 9.92 mmol) to maintain reflux. After 1 h, the reaction was quenched by successive addition of 1 ml of $H_2O$, 1 ml of 15% NaOH, and 3 ml of $H_2O$. The resulting granular salts were filtered and washed with $Et_2O$. After removing the solvent, the compound was obtained as an oil, and used without further purification (2.25 g, 60%). $^1$H NMR (500 MHz, $CDCl_3$) δ: 3.73 (t, 2H), 2.26-2.15 (m,

2H), 1.87 (m, 2H); $^{13}$C NMR (125 MHz. $CDCl_3$) 62.70, 29.08, 28.91, 28.74, 24.67.

**3-perfluorohexyl-1-iodopropane (8).**

The phosphorous pentaoxide (8 g) was added to phosphoric acid (85%, 17 ml) in a 50 ml round-bottom flask and then the mixture was cooled to 0 °C. The KI (3.3 g, 19.87 mmol) was first added, followed immediately by 3-perfluorohexyl-1-propanol (1.7 ml, 7.32 mmol). The mixture was heated at 120 °C for 5 h. At ambient temperature, 20 ml of water was added, and the resulting brown solution was extracted with $Et_2O$ (4 x 25 ml). The organic layer was washed with 0.1 M thiosulfate (3 x 25 ml), then dried over $Na_2SO_4$. After removing the solvent, the iodo derivative was obtained as an oil, and used without further purification (2.85 g, 80%). $^1$H NMR (500 MHz, $CDCl_3$) δ 3.25 (t, 2H), 2.22 (m, 2H), 2.14 (m, 2H); $^{13}$C NMR (125 MHz, $CDCl_3$) 32.53, 32.36. 32.19, 24.70, 4.14;

**Synthesis of Nitrovanillin**

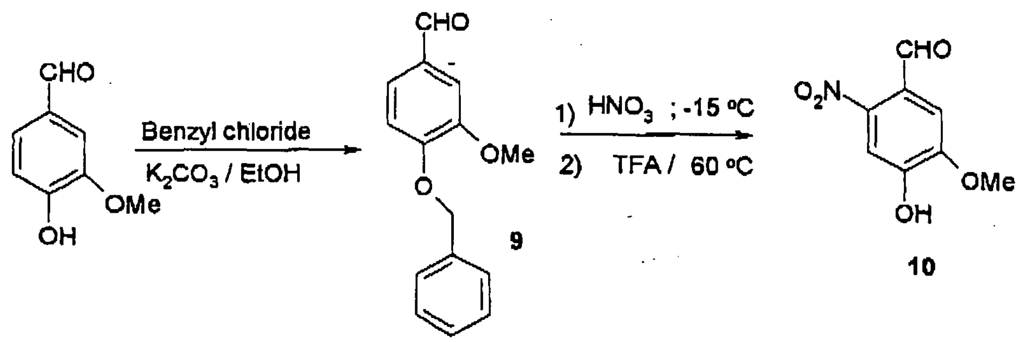


**4-Benzyloxy-3-methoxybenzaldehyde (9)[3]**

To a stirred solution of vanillin (4 g, 26.31 mmol) in ethanol (20 ml) were added $K_2CO_3$ (5 g. 36.18 mmol) and benzyl chloride (3.4 ml. 29.54 mmol), and the mixture was maintained under a nitrogen atmosphere overnight. The solution was filtered through celite, washed with DCM (3 x 25 ml), and then concentrated under vacuum. The oily residue was dissolved in 50 ml DCM, washed with 5% NaOH solution, dried over $K_2CO_8$, concentrated, and crystallised from EtOH. (4.90 g, 77%, mp 61-62 °C) $^1$H NMR (300 MHz, CDCl3) δ 9.82 (s, 1H), 7.46-7.30 (m, 7H), 6.97 (d, 1H), 5.24 (s. 2H), 3.93 (s. 3H); $^{13}$C NMR, (75 MHz, $CDCl_3$) 191.37, 153.98. 150,45, 136.40, 130.67. 129.15, 128.64. 127.63, 127.06, 112.75, 109.69. 71.25, 56.46; MS (EI$^+$) 242 (M$^+$, 28), 151 (8), 91 (100), 65 (28).

**4,5-Hydroxymethoxy-2-nitrobenzaldebyde (10)[4]**

The benzylated vanillin, 9 (1g, 4.13 mmol) and 1.2-dichloroethane (5 ml) were cooled to -30 °C under argon. Fuming nitric acid (2 ml) was added slowly, and the temperature was maintained at ca. -15 °C for three hours. The reaction mixture was poured into water and extracted with ethyl acetate. Removal of the solvent gave a bright yellow solid that was stirred at 60 °C in trifluoroacetic acid (10 ml) for 1 h. Diluted with water (10 ml), neutralised with $NaHCO_3$ and extracted with ethyl acetate. The solvent was removed and the crude product was recrystallised from 95% EtOH to give the product as a dark yellow solid. (0.61 g, 75%, mp 205-207 °C) $^1$H NMR (300 MHz, CDCl3) δ 10.40 (s, 1H), 7.67 (s, 1H), 7.45 (s, 1H), 6.25 (s, 1H), 4.06 (s, 3H); MS (FAB) 197 (M+, 85), 179 (20), 167 (8), 82 (35): Anal. calcd. for $C_8H_7NO_5$: C 48.74%, H 3.58%, N 7.10% found C 48.50%, H 3.45%, N 7.10%.

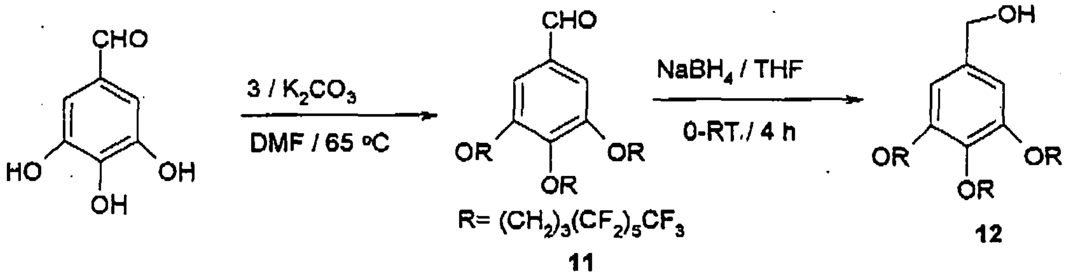


11

12

3,4,5-Tris(1H,1H,2H,2H,3H,3H-perfluoronon-1-yloxy)benzaldehycle (11)

To a mixture of $K_2CO_3$ (0.7 g, 5.06 mmol) and anhydrous DMF (12 ml) under $N_2$ was added 3,4,5-trihydroxybenzaldebyde (0.1 g, 0.58 mmol). 3-perfluorohexyl-1-iodo Propane (1.0 g, 2.04 mmol) was added at 65 °C and the mixture was stirred at 65 °C for overnight. The mixture was poured into cold water (50 m)) and was acidified with concentrated HCl. The aqueous mixture was extracted with $Et_3O$ (3 x 25 ml), washed with water (2 x 50 ml), dried over $MgSO_4$, and the solvent was removed in vacuo. Purification by flash chromatography with ethyl acetate /$^n$hexane (30 / 70) as eluent gave the product as a white solid. (0.53 g, 74%) $^1$H NMR (500 MHz, $CDCl_3$) δ 9.85 (s, 1H), 7.22 (s, 2H), 4.15 (t, J=5.98Hz, 4H), 4.1 (t, J=5.87, 2H), 2.38.2.28 (m, 6H), 2.19-2.13 (m, 4H), 2.06-2.03 (m, 2H); $^{13}$C NMR (75 MHz. $CDCl_3$) 191.13, 153.33, 143.25, 132.46, 108.40, 72.36, 68.05, 28.46, 28.17, 27.87, 21.87, 20.96; MS (FAB) 1235 ($[M+H]^+$, 100), 874 (10); Anal. calcd. for $C_{34}H_{21}F_{19}O_4$: C 33.06%, H 1.71% found C 33.25%, H 1.9%.

**3,4,5-Tris(1H,1H,2H,2H,3H,3H-perfluorononan-1-yloxy)benzylalcohol (12)**

To an ice-cooled solution of 3,4.5-Tris(1H,1H,2H,2H,3H,3H-perfluorononan-1-yloxy)henzaldehyde (1.7 g, 1.37 mmol) in anhydrous THF (20 ml) $NaBH_4$ (0.11 g, 2.88 mmol) was added and the mixture was stirred at 0-RT for 4 h. The reaction was quenched by addition of water (20 ml). The organic layer was separated and the aqueous layer was extracted with EtOAc (3 x 50 ml), and the combined organics were dried over anhydrous $Na_2SO_4$. After filtration, the solvent removed under reduced pressure to give a while solid which was used without further purification. (1.19 g, 70%) $^1$H NMR (500 MHz, $CDCl_3$) δ 6.61 (s, 2H), 4.42 (s, 2H), 4.07 (t, J=5.9 Hz, 4H), 3.99 (t, J=5.86 Hz, 2H). 2.37-2.26 (m, 6H), 2.15-2.09 (m, 4H), 2.03-1.98 (m, 2H); $^{13}$C NMR (125 MHz, $CDCl_3$) 151.21. 135.62, 135.44, 104.07, 70.40, 66.11. 63.74, 26.69, 26.40, 26.11, 19.94, 19.23; MS (FAB) 1236 ($M^+$, 100), 859 (20). 496 (10)108 (100).

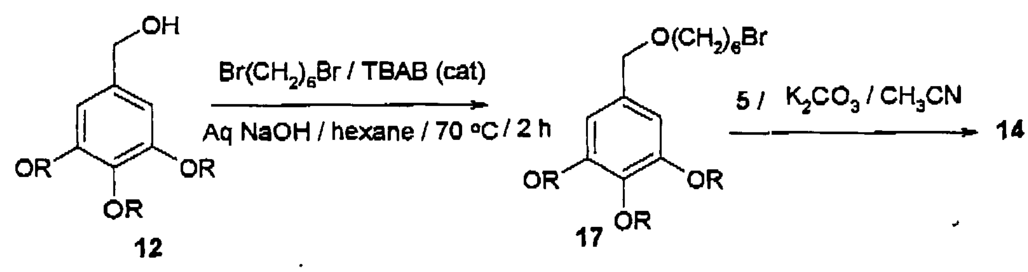


12

17

14

**1-(6- Bromo-hexyloxymethyl)-3,4, 5-tris(1H, 1H,2H,2H, 3H, 3H)-perfluorononan-1-yloxy)b enzene (17)**

50% aqueous NaOH solution (0.7 g), TBAB (12 mg), and hexane (1.6 ml) were placed in a round bottom flask. 3,4,5 Tris(1H,1H,2H,2H,3H,3H-perfluorononan-1-yloxy)benzylalcohol (0.5 g, 0.4 mmol) and 1,6-dibromohexane (0.15 ml, 0.97 mmol) were added with efficient stirring. It was kept at room temperature for 10 min and then refluxed for 2 h. It was cooled to RT and 10 ml of water was added. The organic layer, which was separated by means of separatory funnel, was dried over anhydrous $Na_2SO_4$. Purification by flash chromatography with ethyl acetate / $^n$hexane (20 / 80) as eluent gave the product as a white solid. (0.42 g, 75%) $^1$H NMR (300 MHz, $CDCl_3$) δ 6.56 (s. 2H), 4.40 (s, 2H), 4.07 (t, J=5.83, 4H), 3.98 (t, J=5.84, 2H), :3.47 (t, J=6.50, 2H), 3.41 (t, J=6.76, 2H), 2.34-2.23 (m, 6H), 2.17-2.07 (m, 6H), 2.05-1.96 (m, 2H), 1.91-1.82 (m, 2H), 1.68-1.59 (m, 2H), 1.51-1.39 (m, 4H); $^{13}$C NMR (75 MHz. $CDCl_3$) 152.53,

136.83; 134.79, 106.25. 72-91, 71.82, 70.64, 70.48, 67.52, 33.86, :32.72. 29.77, 29.57, 28.18, 27.99, 27.89. 27.59, 26.22, 25.46, 21.41, 20.70: MS (ES+) 1421 & 1423 ([M+Na]+, 50). 1398 & 1400 ($M^+$, 42); Anal. calcd. for $C_{40}H_{34}F_{39}O_4Br$: C 34.31%, H 2.45%, Br 5.71% found C 34.35 %, H 2.7 %, Br 5.45 %. 14

To a mixture of $K_2CO_3$ (0.2 g. 1.44 mmol) and anhydrous acetonitrile (20 ml) under $N_2$ was added 4,5-Hydroxymetboxy-2-nitrobenzaldehyde, 5 (80 mg, 0.4 mmol). 1-(6-Bromo-hexyloxymethyl)-3,4,5-tris(1H,1,H,2H,2H,3H, 3H)-perfluorononan-1-yloxy) benzene, 12 (0.3 g, 0.23 mmod was added at 70 °C and the mixture was refluxed for overnight. The mixture was filtered and the filtrate was concentrated in vacuo to remove the solvent. Purification by flash chromatography with ethyl acetate / $^n$hexane (30 70) as eluent gave the product as a yellow sticky solid. (0.35 g, 65%) $^1$H NMR (500 MHz, $CDCl_3$) δ 10.44 (s, 1H), 7.58 (s, 1H), 7.41 (s, 1H), 6.56 (s, 2H), 4.40 (s, 2H), 4.14 (t. J=6.68, 2H), 4.07 (t, J=5.83, 4H), 4.00 (s, 3H), 3.98 (t, J=5.85, 2H), 3.48 (t, J=6.48), 2.35-2.26 (m, 6H), 2.13-2.08 (m, 4H), 2.04-1.99 (m, 2H), 1.93-1.90 (m, 2H), 1.66 (m, 2H), 1.54-1.47 (m, 4H); $^{13}$C NMR (75 MHz, $CDCl_3$) 188.16, 153.81, 1.52.88, 152.36, 144.26, 137.16, 135.15, 135.10, 125.69, 110.23, 108.29, 106.67, 106.58, 73.30, 72.17, 70.99, 70.90.70.19, 67.87, 57.07, 34.23, 33.07, 30.12. 30.04, 29.91, 29.13, 28.52, 28.30, 28.22, 27.93, 26.57, 26.39, 26.15, 25.81, 21.76, 21.06; MS (ES+) 1538 ([M+Na]$^+$, 80), 1219 (100), 402 (65), 381 (60); Anal. calcd. for $C_{48}H_{40}F_{39}O_9N$: C 38.01%, H 2.66%, N 0.92% found C 37.95%. H 2.60%, N 0.85%.

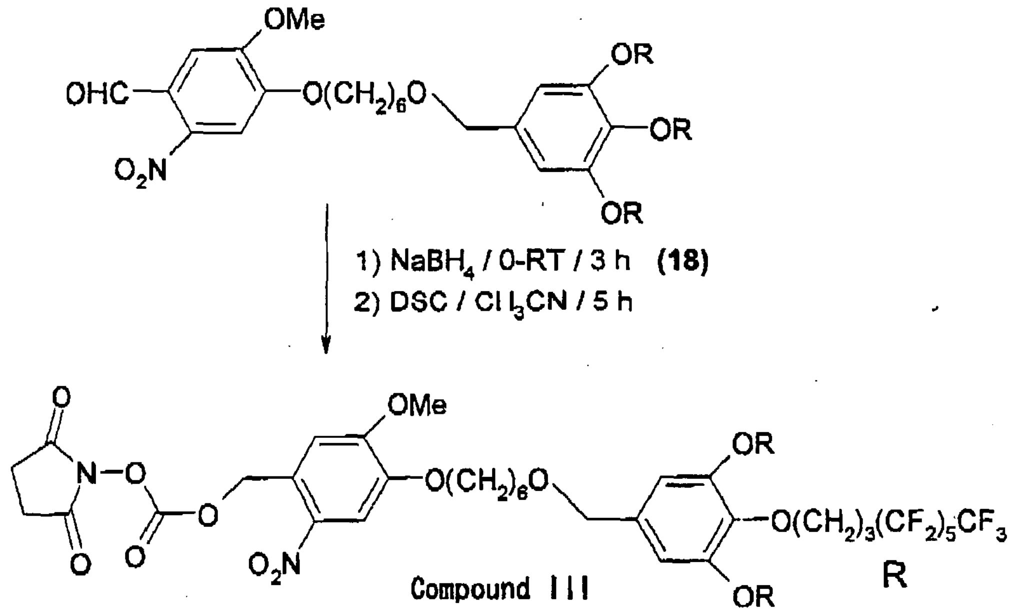


18

To an ice-cooled solution of 14 (0.42 g, 0.27 mmol) in anhydrous THF (10 ml) $NaBH_4$ (25 mg, 0.65 mmol) was added and the mixture was stirred at 0-RT for 4 h. The reaction was quenched by addition of water (10 ml). The organic layer was separated and the aqueous layer was extracted with EtOAc (3 x 25 ml), and the combined organics were dried over anhydrous $Na_2SO_4$. After filtration, the solvent removed under reduced pressure to give a white solid which was purified by flash chromatography with ethyl acetate / $^n$hexane (40 / 60) as eluent to gave the product as a pale yellow sticky solid. (0.41 g, 72%) $^1$H NMR (500 MHz, $CDCl_3$) δ 7.69 (s, 1H), 7.15 (s, 1H), 6.60 (s, 2H), 4.95 (s, 2H), 4.62 (s, 2H), 4.07 (m, 6H), 4.06 (t, 2H), 3.99 (s, 3H), 3.48 (t, J=6.73, 2H), 2.35-2.26 (m, 6H), 2.13-2.08 (m, 4H), 2.04-1.99 (m, 2H), 1.93-1.90 (m. 2H), 1.68-1.64 (m, 2H), 1.54-1.47 (m, 4H): $^{13}$C NMR (75 MHz, $CDCl_3$) 154.60, 152.87, 147.83, 135.14, 132.51, 111.56, 109.60, 106.60, 73.27, 72.17, 70.94, 69.78, 67.85, 63.28. 56.81, 30.04, 29.25, 28.23, 27.94, 26.40. 26.18, 21.05; MS (ES+) 1540 ([M+Na]$^+$, 100), 1219 (60), 319 (30); Anal. calcd. for $C_{48}H_{42}F_{39}O_9N$: C 37.96%. H 2.79%, N 0.92% found C 37.75%, H 2.75%, N 0.90%.

**Compound III**

To a stirred solution of, 18 (0.17 g. 0.11 mmol) in anhydrous $CH_3CN$ (8 ml), under argon atmosphere, was added *N,N*-disuccinimidyl carbonate (40 mg, 0.15 mmol) followed by $Et_3N$ (0.05 ml, 0.35 mmol) The solution was stirred for an additional 5 h and then the solvent was removed in vacuo. The residue was purified by silica gel column using

DCM / EtOAc (96 / 4), gave the compound as pale yellow thick oily solid. (0.18 g, 68%) $^1$H NMR (300 MHz, $CDCl_3$) δ: 7.74 (s,1H), 7.03 (s,1H), 6.56 (s, 2H), 5.78 (s, 2H). 4.40 (s, 2H), 4.07 (m, 6H), 4.03 (s, 3H). 3.97 (t, J=5.50 Hz, 2H). 3.48 (t, J=6.45, 2H), 2.85 (s, 4H), 2.35-2.26 (m, 3H), 2.13-2.08 (m, 4H). 2.04-1.99 (m, 2H), 1.91 (m, 2H), 1.66 (m, 2H), 1.54-1.47 (m, 4H); $^{13}$C NMR (75 MHz, $CDCl_3$ 168.87. 154.86. 152.87, 151.83, 148.40, 139.44, 137.16, 135.13, 125.55, 109.62, 109.20, 106.63, 73.28, 72.16, 70.95, 69.81, 69.62, 67.85, 57.01, 30.03, 29.21, 28.53, 28.24, 27.94, 26.38, 26.19, 25.85, 25.57, 21.76, 21.05; MS (ES+) 1681 ([M+Na]$^+$, 65), 1484 (35), 1219 (60), 419 (100), 282 (100), 235 (55); Anal. calcd. for $C_{59}H_{45}F_{39}O_{19}N_2$: C 38.35%, H 2.73%, N 1.69% found C 38.45%, H 2.85%, N 1.7%.

The following Embodiments provide further details of the present invention. However, it should be noted that the present invention is in no way limited to the specifics of these Embodiments. Many variations and modifications to the embodiment can be made without departing from the scope of the appended claims. First embodiment

(Synthesis of the following compound 1)

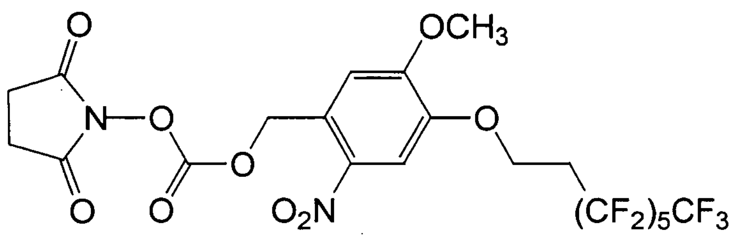

The compound 1 was prepared in accordance with the synthesis set out in FIG. 1 and its reaction steps 1-4.

The spectral data of $^1$H NMR, $^{13}$C NMR, MS, and the like for the material (white solid) synthesized in reaction step (1) are as follows, and this material was identified as 4-(1H, 1H, 2H, 2H-perfluorooctyloxy)-3-methoxy-benzaldehyde (a yield of 30%).

$^1$H NMR (300 MHz, CDCl3) 9.87 (s, 1H, Ar-H), 7.48-7.43 (m, 2H, Ar-H), 6.99 (d, 1H, Ar-H), 4.43-4.38 (t, 2H), 3.93 (s, 3H), 2.81-2.69 (m, 2H); $^{13}$C NMR (75 MHz, CDCl3) 191.28, 153.26, 150.35, 131.28, 126.90, 1112.34, 110.02, 61.54, 56.47, 31 .82, 31.54, 31.25; MS (ES+) 499 ([M+H]$^+$, 55), 454 (20), 391 9220, 279 (5), 241 (5).

The spectral data of $^1$H NMR, $^{13}$C NMR, MS, elemental analysis, and the like for the material (yellow solid) synthesized in reaction step (2) are as follows, and this material was identified as 2-nitro-4-(1H, 1H, 2H, 2H-perfluorooctyloxy)-5-methoxy-benzaldehyde (a yield of 80%).

$^1$H NMR (300 MHz, $CDCl_3$) 7.64 (s, 1H, Ar-H), 7.43 (s, 1H, Ar-H), 4.47-4.43 (t, 2H), 4.01 (s, 3H), 2.86-2.69 (m, 2H);$^{13}$C NMR (75 MHz, CDCl3) 188.08, 154.01, 1 51.17, 126.77, 110.71, 108.91, 91.23, 62.28, 57.17, 31.78, 31.48, 31.21; MS (ES+) 544 ([M+H]$^+$, 100), 514 (15), 410 (15), 282 (10), 178 (10); Anal. Calcd. For $C_{16}H_{10}F_{13}O_5N$: C 35.35%, H 1.85%, N 2.57% found C 35.35%, H1.95%, N 2.75%.

The spectral data of $^1$H NMR and $^{13}$C NMR for the material (solid) synthesized in reaction step (3) are as follows, and this material was identified as 2-nitro-4-(1H, 1H, 2H, 2H-perfluorooctyloxy)-5-methoxy-benzyl alcohol (a yield of 70%).

1H NMR (300 MHz, CDCl3) 7.74 (s, 1H, Ar-H), 7.22 (s, 1H, Ar-H), 4.98 (s, 2H), 4.40-4.35 (t, 2H), 3.99 (s, 3H), 2.82-2.65 (m, 2H), 2.62 (br.s, 1H); $^{13}$C NMR (75 MHz, $CDCl_3$) 154.80, 146.79, 139.89, 133.75, 111.82, 110.56, 63.18, 56.90, 31.80 , 31.54, 31.26

. The spectral data of $^1$H NMR, $^{13}$C NMR, MS, elemental analysis, and the like for the material (pale yellow solid) synthesized in reaction step (4) are as follows, and this material was identified as the compound 1 (a yield of 62%).

$^1$H NMR (300 MHz, $CDCl_3$) 7.80 (s, 1H, Ar-H), 7.23 (s, 1H, Ar-H), 5.99 (s, 2H), 4.41-4.37 (t, 2H), 4.05 (s, 3H), 2.87 (s, 4H), 2.80-2.68 (m, 2H); $^{13}$C NMR (75 MHz, $CDCl_3$) 168.88,155.07, 151.81, 147.36,139.23, 126.89, 110.54, 109.49, 69.48 62.09, 57.08, 31.76, 31.51, 31.23, 25.86; MS(FAB) 686 ([M]$^+$, 8), 528 (100); HRMS (ES+) Calcd. For $C_{21}H_{15}N_2O_9F_{13}Na$ 709.0468, found 709.0473; Anal. Calcd. For $C_{21}H_{15}N_2O_9F_{13}$: C 36.73%, H 2.20%, N 4.08% found C 36.45%, H 2.35%, N 4.25%.

Second embodiment

A gold film was formed on a surface of a silicon substrate by sputtering. Separately, a thiol solution was prepared by dissolving 1 mM of 11-aminodecanethiol in ethanol. Next, the substrate provided with the gold film thereon was immersed into the thiol solution at room temperature for 12 hours. The substrate was then rinsed with ethanol and

subsequently dried under nitrogen flow. This resulted in a SAM film of 11-aminodecanethiol being formed on the gold surface of the substrate.

Subsequently, 150 mg of the compound 1 as synthesized and purified in Embodiment 1 was dissolved in 100 m1 of anhydrous dichloromethane to form a solution at a concentration of approximately 2 mM, and 2 ml of triethylamine was dissolved in the solution thus formed, thereby preparing a solution of compound 1. Next, the above-described substrate (hereinafter referred to as the "amino-surface substrate") having amino functional groups on the surface thereof was immersed in the solution thus prepared at room temperature for half a day, which allows the reaction between the succinic imide ester of compound 1 and the amino group of the amino-surface substrate to proceed. This reaction is illustrated in FIGS. 2(1) and 2(2). After this reaction, the substrate was rinsed with dichloromethane, followed by drying by flowing nitrogen gas. A fluorinated chain modified SAM composed of molecules each having the structure shown in FIG. 2(2) was thus formed on the substrate surface.

The contact angle of the SAM (monomolecular film) surface thus formed was measured, and the advance angle (water) was approximately 90 to 100°. The variation in the measured angle is believed to be due to variations in the molecular coating state of the fluorinated chain modified SAM.

The fluorinated chain modified SAM surface was then irradiated with UV having a wavelength of 365 nm through a photomask for varying time periods (1 minute, 5 minutes, 10 minutes, etc.). The UV irradiation energy distribution used is illustrated in FIG. 3. The UV irradiation energy was approximately 30 $mW/cm^2$. The UV irradiation resulted in the decomposition reaction shown in FIGS. 2(2) and 2(3) to occur, and as a result, a SAM having amino groups represented by the structure shown in FIG. 2(3) was formed on the surface by decomposition.

After the UV irradiation, the substrate surface was rinsed with ethanol, and the change in the surface contact angle (advance angle (water)) at four different locations where the SAM had been irradiated was measured. The results of the measurement are listed in Table 1. The followings are clarified by the result shown in Table 1. The contact angle is reduced to approximately 50° when the SAM is irradiated for 1 to 5 minutes suggesting that the decomposition of the coupled SAM is substantially completed during this time period. Since the contact angle of the SAM surface when covered only with 11-aminodecanethiol was measured to be approximately 43°, it is believed that most of the fluorinated chains were removed by the irradiation.

In addition, the SAM was immersed in water after the irradiation and rinsing steps and was then pulled out therefrom. The surface of the SAM was then immediately observed. Water droplets were observed to remain where the SAM surface had been irradiated with UV that is the hydrophilic property was confirmed. On the other hand, the SAM surface repelled water at locations where it had been masked from the UV radiation that is the hydrophobic property was maintained.

Comparative example

The silicon substrate coated with a solid film used as a starting substrate in Embodiment 2 was immersed in a dichloromethane solution containing 1 mM of $HS(CH_2)_2(CF_2)_9CF_3$. Then UV irradiation was performed in a manner similar to that in Embodiment 2, and the wettability of the surface was analyzed. As a result, the contact angle before UV treatment was 110° with respect to water, and the contact angle after UV irradiation for 5 minutes was approximately 109°, that is essentially unchanged. Accordingly, it was confirmed that a SAM formed from $HS(CH)_2(CF_2)_9$ was hardly decomposed by UV light (see the right-hand column of Table 1).

Table 1

| | Embodiment 2 | | | | Comparative Example |
|---|---|---|---|---|---|
| | Compound 1 | | | | $CF_3(CF_2)_9(CH_2)_2SH$ |
| Area Measured | 1 | 2 | 3 | 4 | |
| | C.A (°) | C.A (°) | C.A (°) | C.A (°) | C.A(°) |
| UV 0 min | 95 | 97 | 102 | 98 | 110 |
| UV 1 min | 67 | 67.8 | 66 | 69 | 109 |
| UV 5 min | 53.1 | 53 | 52.6 | 53.1 | 109 |
| UV 10 min | 50 | 49.5 | 49.7 | 50.9 | 108 |
| UV 20 min | 50.5 | 50.1 | 50.8 | 51.4 | 104 |
| UV 30 min | 48.5 | 49.7 | 48.8 | 48.9 | 105 |

Table 1 (continued)

| | Embodiment 2 | | | | Comparative Example |
|---|---|---|---|---|---|
| | Compound 1 | | | | $CF_3(CF_2)_9(CH_2)_2SH$ |
| Area Measured | 1 | 2 | 3 | 4 | |
| | C.A (°) | C.A (°) | C.A (°) | C.A (°) | C.A(°) |
| UV 60 min | 49.2 | 49.7 | 50.1 | 51.1 | 103 |

From the above description, it can be understood that the present invention provides a self-assembled monolayer (SAM) material which can form a monomolecular film on a substrate and which can be photo-patterned by a relatively short exposure to low energy UV irradiation. The SAM material provided can be applied to a variety of different types of substrates by means of suitable coupling compounds. Prior to irradiation, the SAM material is hydrophobic and/or lipophobic. Irradiation however cleaves away the structural component responsible for this property leaving behind a functional group which is hydrophilic. Irradiation of the SAM material therefore substantially alters the surface property of the SAM-coated substrate.

Embodiment 3

In the embodiment, wettability of the solid-phase surface is controlled by ejecting solution of the compound described as the above Formula (II) (called as the compound (II)). The compound (II) includes a N-hydroxy succinic imide group as the structure X, a structure having photic-decomposable property as the structure Y, a fluorinated chain as the structure Z, which changes the solid-state property of the solid-phase surface to hydrophobic and/or lipophobic.

(Preprocessing the surface of the substrate)

First, as shown in FIG. 5, 11-amino dodecanethiol is fixed to the substrate surface. This compound is capable of coupling with a N-hydroxy succinic imide of the compound (II). The substrate is silicon and coated with a gold film by evaporation in advance. The gold film is also formed by sputtering.

11-amino dodecanethiol is dissolved into ethanol to be 1mM and the substrate is immersed into this solution. The substrate was then rinsed with ethanol and subsequently dried under nitrogen flow. The substrate surface is covered with an amino group and turned to be hydrophilic.

(Fixing the compound (II))

Next, 150 mg of the compound I is dissolved into 100mL of anhydrous DMSO (dimethyl sulfoxide) to be about 2mM solution and 2mL of trimethyl amine is added.

As shown in FIG. 6, after deaerating solution, the solution is flown into an ink reservoir of ink-jet type ejecting device and ejected on the given location of the above amino substrate surface.

After this ejection, the substrate was rinsed with DMSO, followed by drying by flowing nitrogen gas. As the result, the compound (II) is fixed only onto a region where droplets are ejected and hydrophobic and/or liophobic property is given by fluorinated chain represented as CF. On the other hand, a region where droplets are not ejected shows hydrophilic since the amino group remains exposed.

Water is developed onto the surface and observed by a microscope. The observed results are shown in FIG. 7 to FIG. 10. According to FIG. 7 and 8, it is confirmed that water is shed in the region where the solution of the compound (II) is ejected. Further, in FIG. 9 and FIG. 10, it is confirmed that a polar organic solvent such as ethanol is also shed showing printed pattern and the region where the solution of the compound (II) is ejected and a liquid is not attached to the substrate surface.

(Fixing the amino protective group)

Next, as shown in FIG. 11, DMSO solution such as 9-Fluoprenylmethyl succinimidyl carbonate, for example, is ejected by the ink jet method or the like in the region where the compound (II) is not ejected. Otherwise, the substrate where the compound (II) is patterned is immersed into the solution so that an amino protective group is reacted and fixed. As the amino protective group, benzyl N-succinimidyl carbonate, di-tert-butyldicarbamate and N-Boc imidasole are also cited.

(Decomposing the compound (II))

Subsequently, as shown in FIG. 12, 308 nm UV is irradiated onto the substrate surface. The compound (II) is dissolved thereby with reaction shown in FIG. 4 and fluorinated chain represented as CF is removed from the substrate surface exposing an amino group again and showing hydrophilic property in this region. Then, the active amino group is reacted with a solution where NHS ester (NHS-LC-LC-Biotin, a reagent produced by Pierce Inc.) is dissolved forming a micro pattern covered with the biotin. Further, on this pattern, HRP(horseradish peroxidase) enzyme molecule conjugated with streptavidin is fixed for example, easily forming a micro pattern of an enzyme monomolecular film.

## Claims

1. A molecule comprising a structural component (A) which decomposes when irradiated with UV light having a wavelength in the range 254-400 nm, and a structural component (B) which is hydrophobic and/or lipophobic.

2. The molecule according to Claim 1, wherein the structural component (A) is an o-nitrobenzyl ester.

3. The molecule according to Claim 2, wherein the terminal group bonded to the benzyl group of the o-nitrobenzyl ester is a succinic imide.

4. The molecule according to any of claims 1 to 3, wherein the structural canponent (B) comprises a fluorinated chain.

5. The molecule according to Claim 4, wherein the fluorinated chain is saturated.

6. The molecule according to Claim 4 or Claim 5, wherein the fluorinated chain is branched and/or perfluorinated.

7. The molecule according to any of claims 1 to 6, having the following general Formula (1):

[Formula 1]

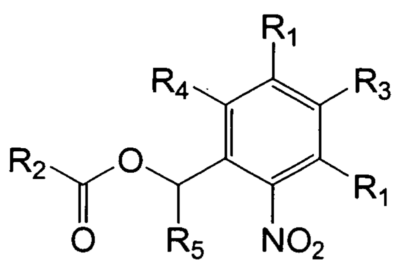

(I)

wherein:

$R_1$ independently represents a hydrogen atom, $-OR_6$ (wherein $R_6$ is an alkyl chain having a carbon number of 1 to 10), $-NH(CO)R_7$ (wherein $R_7$ represents an alkyl chain having a carbon number of 1 to 10, or an alkyl fluoride chain having a carbon number of 1 to 10), $N(R_8)_2$ ($R_8$ represents an alkyl chain having a carbon number of 1 to 5) or-$S(R_9)$ (wherein $R_9$ represents an alkyl chain having a carbon number of 1 to 10).
$R_2$ represents an N-hydroxy succinic imide group (optionally substituted with a sulfonyl group);
$R_3$ represents $-X_2-(CH_2)_n-X_1$, wherein $X_2$ represents $-CH_2-$ or -O-, n is 0 or an integer of 1 to 10, and $X_1$ represents -OZ, -Z or

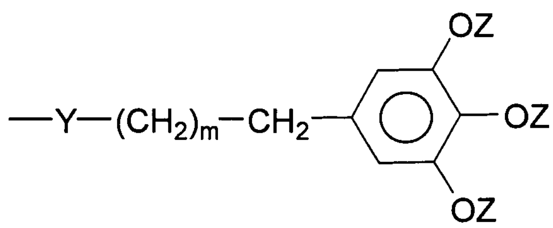

wherein:

Y represents $-(CH_2)-$ or -O-, Z represents a fluoroalkyl group having a carbon number of 1 to 20 and m represents 0 or an integer of 1 to 10;
$R_4$ represents $-NO_2$ or a hydrogen atom; and
$R_5$ is a hydrogen atom or an alkyl group having a carbon number of 1 to 10.

**8.** The molecule according to Claim 7, wherein $R_1$ is $-OCH_3$.

**9.** The molecule according to Claim 7 or Claim 8, wherein Z is $-(CH_2)_m(CF_2)_pF$ or a branched chain isomer thereof wherein m is as defined above and p is 0 or an integer of 1 to 9.

**10.** The molecule according to any of claims 1 to 9, which is the following compound:

[Formula 2]

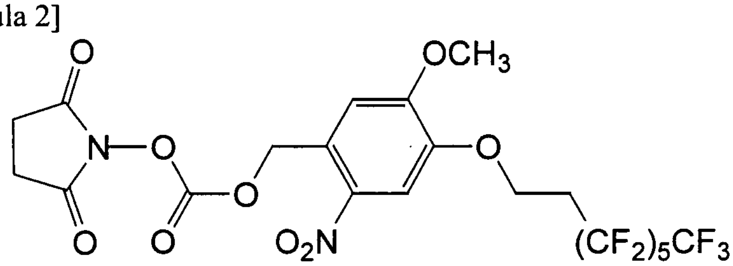

**11.** A monomolecular film coated substrate, the film being obtainable by coating, on a substrate, molecules comprising a structural component (B) which is hydrophobic and/or lipophobic, and a structural component (A) which decomposes when irradiated with UV light having a wavelength in the range 254-400 nm to cleave away a part of the molecule comprising the structural component (B) leaving a residual hydrophilic structural component (C).

**12.** The monomolecular film coated substrate according to Claim 11, wherein the structural component (A) is an o-nitrobenzyl ester.

**13.** The monomolecular film coated substrate according to claim 11 or Claim 12, wherein the structural component (B) comprises a fluorinated chain.

**14.** The monomolecular film coated substrate according to Claim 13, wherein the fluorinated chain is saturated.

**15.** The monomolecular film coated substrate according to Claim 13 or Claim 14, wherein the fluorinated chain is branched and/or perfluorinated.

**16.** The monomolecular film coated substrate according to any of Claims 11 to 15, wherein the hydrophilic structural component (C) comprises an amine group or a hydroxyl group.

**17.** The monomolecular film coated substrate according to any of Claims 11 to 16 obtainable by coating a substrate with a monomolecular film of a coupling compound (D) which comprises a hydrophilic moiety and subsequently coating the thus formed substrate coated with a monomolecular film of compound (D) having a hydrophilic moiety with a molecule according to any of Claims 1 to 10 and subjecting the thus coated substrate to conditions suitable to covalently bond the molecule according to any of Claims 1 to 10 to the coupling compound (D).

**18.** The monomolecular film coated substrate according to any one of Claims 11 to 17, wherein the substrate is formed from a glass, a metal, a semiconductor or a plastics.

**19.** The monomolecular film coated substrate according to Claim 17 or Claim 18, wherein the hydrophilic moiety of compound (D) is an amine group or a hydroxyl group.

**20.** The monomolecular film coated substrate according to any of Claims 11 to 15, wherein the molecules forming the film are as defined in any of Claims 1 to 10.

**21.** The monomolecular film coated substrate according to any of Claims 11 to 15 or Claim 20, wherein the substrate has a hydrophilic surface prior to coating with the monomolecular film.

**22.** A method of photo-patterning the monomolecular film of the monomolecular film coated substrate as defined in any of Claims 11 to 21 comprising the step of image-wise irradiating the monomolecular film coated substrate with UV light having a wavelength in the range 254-400 nm through a patterned mask to cleave the coated molecules at the structural component (A) thus removing the structural component (B) from the coated film in the irradiated areas converting them from being hydrophobic and/or lipophobic to hydrophilic.

**23.** A method of forming a film pattern comprising; at least a step of ejecting a droplet, which includes a compound represented by the following Formula (0), on a solid-phase surface having a functional moiety;

$$\textbf{X-Y-Z} \qquad (0)$$

where, X represents a structural component that can react with a functional moiety which exists at the solid-phase surface, Y represents a decomposable structural component by itself and Z represents a structural component which is capable of changing a solid-state property on the solid-phase surface or Z represents a reactive structural component.

**24.** The method according to Claim 23, wherein the solid-state property is wettablity.

**25.** The method according to Claim 23 or 24, wherein Z includes a structural component, which contains a group that is selected from any of groups including a saturated or unsaturated alkyl chain optionally having a substituent, a saturated or unsaturated fluorinated chain optionally having a substituent, hydoxyl group, amino group, urethane group, carboxyl group, carbonyl group, urea group, sulfonic group, disulfide group, epoxy group, carbodiimide group, maleimido group, N-hydroxy succinic imide group.

**26.** The method according to any of Claims 23 to 25, wherein X includes a structural component, which contains a group that is selected from any of groups including amino group, urethane group, carboxyl group, carbonyl group, urea group, sulfonic group, disulfide group, epoxy group, carbodiimide group, maleimido group, alkoxy silane, silane halide, and N-hydroxy succinic imide group.

**27.** The method according to any of Claims 23 to 26, wherein Y is a structural component having an optical response property.

**28.** The method according to Claim 23, wherein the compound of Formula (0) is a molecule as defined in Claim 7.

**29.** The method according to any of Claims 23 to 28 further comprising; a step of fixing a compound having a functional moiety, which is capable of bonding to group X, onto the solid-phase surface before ejecting a liquid including a compound represented by the Formula (0) or (I).

**30.** The method according to any of Claims 23 to 29, wherein the method of ejecting a droplet is a ink-jet method.

**31.** The method according to any of Claims 23 to 30, wherein the droplet includes a solvent, which is selected from the group consisting of water, ethanol, DMF, DMSO, HMPA, pyrrolidone group, and dioxane.

**32.** The method according to any of Claims 23 to 31, wherein the method of ejecting a droplet includes a means of controlling to dry the droplet.

**33.** The patterned product obtainable by the methods of any one of Claims 22 to 31.

**34.** A process of preparing a monomolecular film coated substrate, which includes the step of coating the compound according to any one of Claims 1 to 10 on a substrate surface.

**35.** The process of Claim 34, wherein the substrate surface is coated with a coupler compound (D) having a hydrophilic group, prior to the coating with the compound according to any one of Claims 1 to 10, and wherein, after the coating with the compound according to any one of Claims 1 to 10, the coated substrate is subjected to conditions such that the compound according to any one of Claims 1 to 10 reacts with the coupler compound (D) to form a covalent bond.

**36.** The process of Claim 34, wherein no coupler compound is used, and wherein the support surface exhibits a hydrophilic property prior to the coating with the compound according to any one of Claims 1 to 10.

**37.** The molecule according to Claim 4, wherein the fluorinated chain is linear, branced or cyclic and has 1 to 20 carbon atoms and 3 to 41 fluorine atoms.

**38.** The molecule according to Claim 7, wherein in Formula (I):

R1 independently represents a hydrogen atom, $-OR_6$ (wherein $R_6$ is a linear, branched or cyclic alkyl chain having a carbon number of 1 to 10), $-NH(CO)R_7$ (wherein $R_7$ represents a linear, branched or cyclic alkyl chain having a carbon number of 1 to 10, or a linear, branched or cyclic alkyl fluoride chain having a carbon number of 1 to 10 and havinf 3 to 21 fluoride atoms), $N(R_8)_2$ (wherein the individual substituents $R_8$ can be the same or different from each other, and wherein each $R_8$ represents a linear, branched or cyclic alkyl chain having a carbon number of 1 to 5, or wherein the two substituents $R_8$ may be linked together to form a 3 to 11 membered cyclic structure) or-$S(R_9)$ (wherein $R_9$ represents a linear, branched or cyclic alkyl chain having a carbon number of 1 to 10).

$R_2$ represents an N-hydroxy succinic imide-derived group (optionally substituted with a substituent containing a sulfonyl group), which is linked to the ester group via the oxygen atom of the N-hydroxy unit;

$R_3$ represents $-X_2-(CH_2)_n-X_1$, wherein $X_2$ represents $-CH_2-$ or -O-, n is 0 or an integer of 1 to 10, and $X_1$ represents-OZ, -Z or

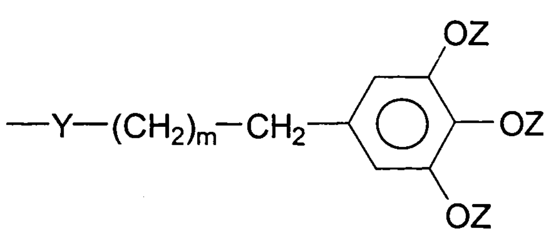

wherein:

Y represents -(CH2)- or -O-, Z represents a linear, branched or cyclic fluoroalkyl group having a carbon number of 1 to 20 and 3 to 41 fluorine atoms, and m represents 0 or an integer of 1 to 10;

$R_4$ represents -NO2 or a hydrogen atom; and

$R_5$ is a hydrogen atom or a linear, branched or cyclic alkyl group having a carbon number of 1 to 10.

**39.** The molecule according to Claim 13, wherein the fluorinated chain is linear, branced or cyclic and has 1 to 10 carbon atoms and 3 to 21 fluorine atoms.

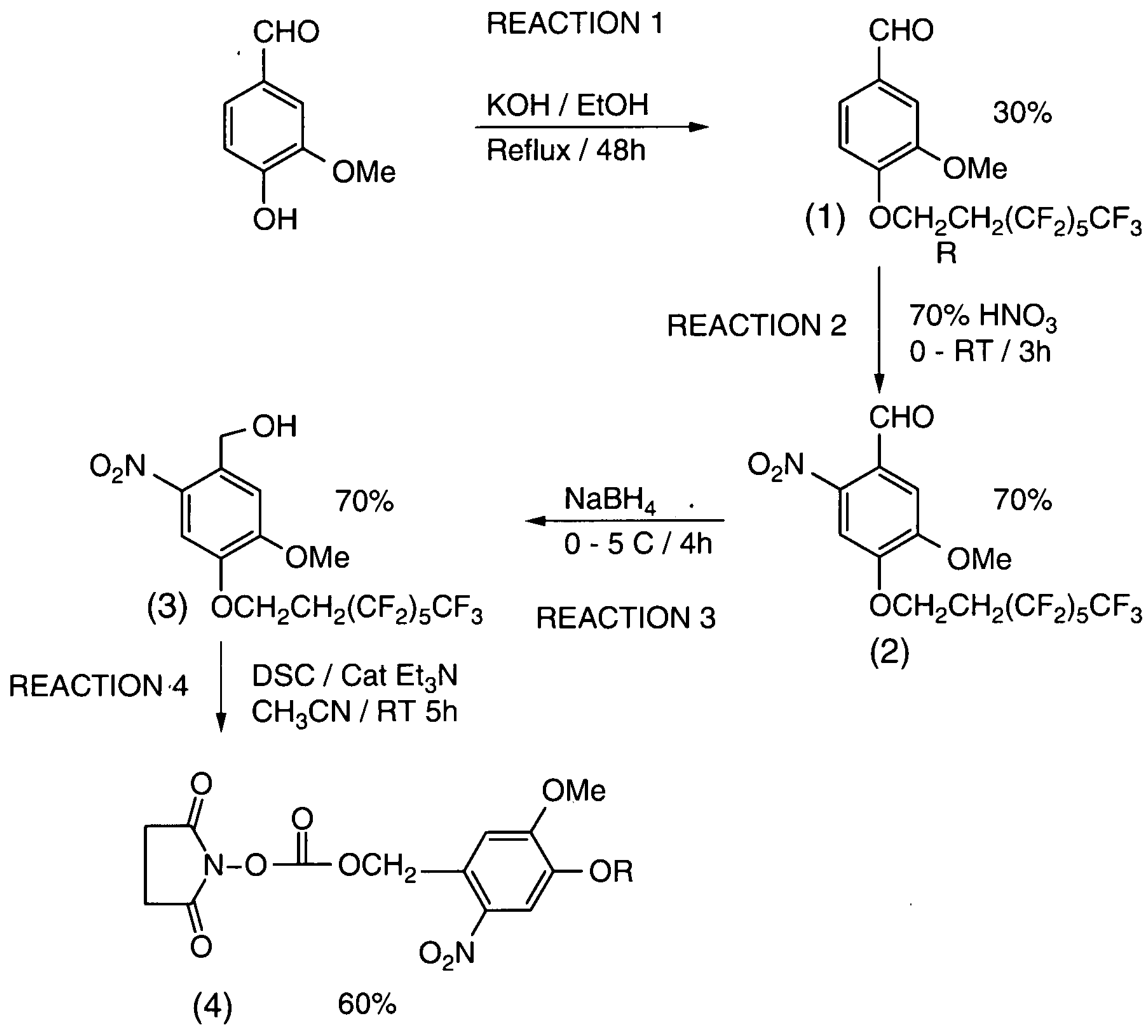
REACTION 1
KOH / EtOH
Reflux / 48h
30%
(1)
REACTION 2
70% HNO3
0 - RT / 3h
70%
(2)
NaBH4
0 - 5 C / 4h
REACTION 3
70%
(3)
REACTION 4
DSC / Cat Et3N
CH3CN / RT 5h
(4)
60%

FIG. 1

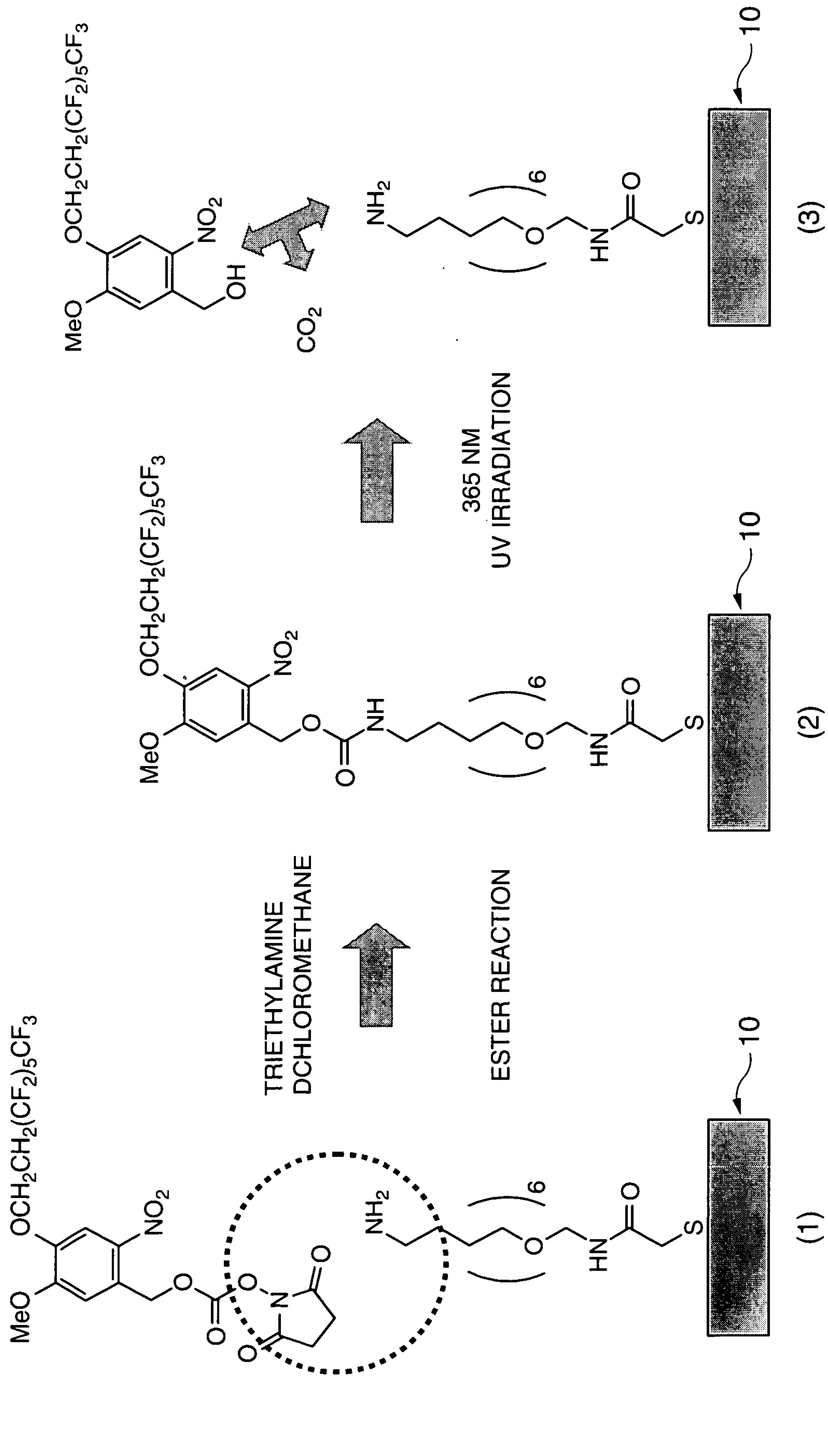
TRIETHYLAMINE
DCHLOROMETHANE
ESTER REACTION
365 NM
UV IRRADIATION
10
(1)
(2)
(3)

FIG. 2

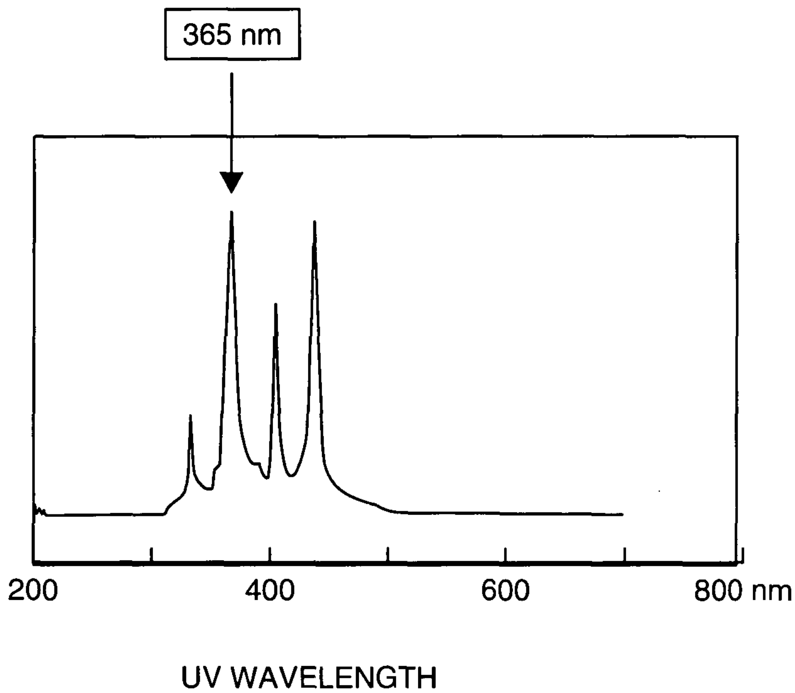


FIG. 3

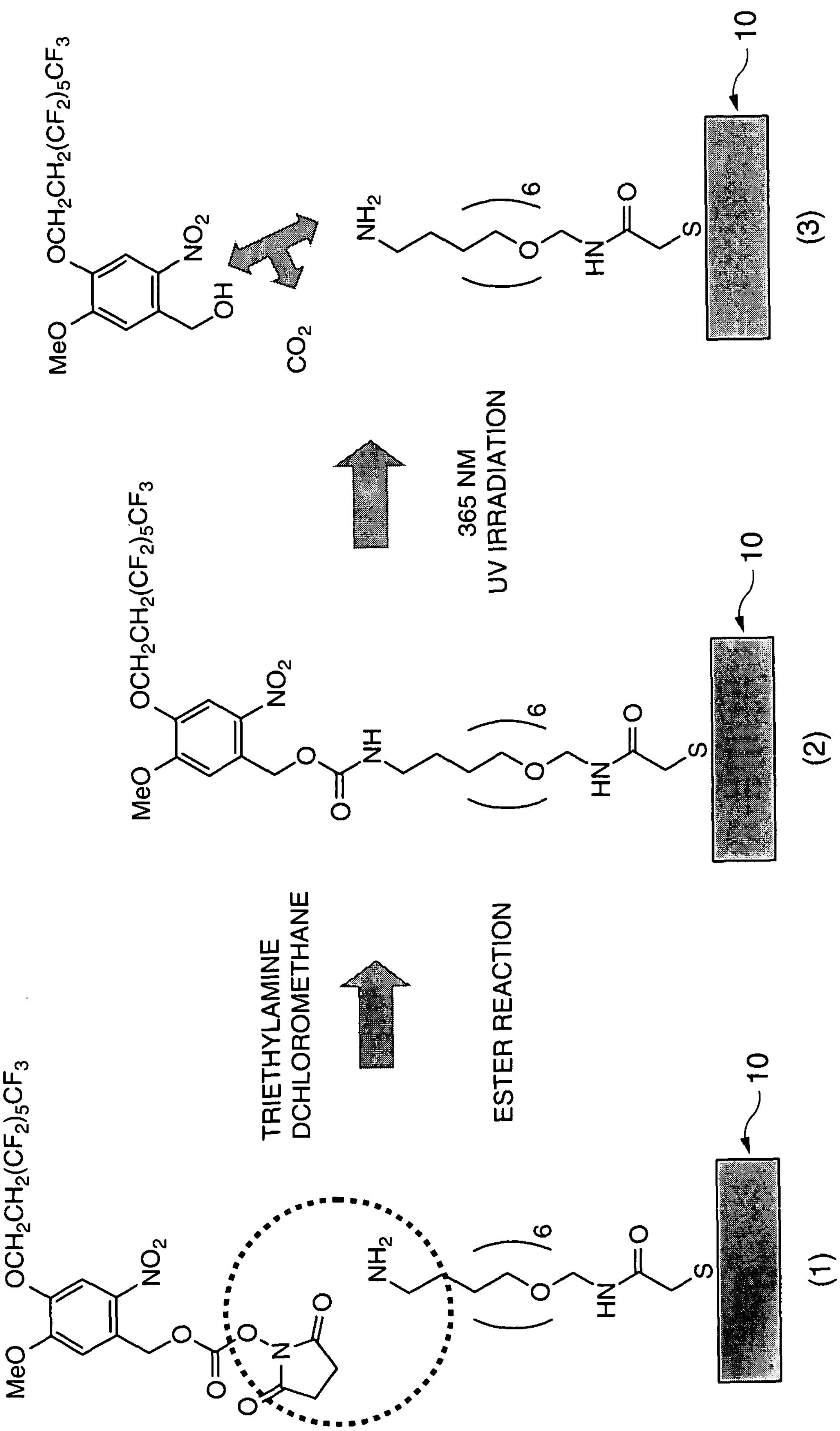
TRIETHYLAMINE
DCHLOROMETHANE
ESTER REACTION
365 NM
UV IRRADIATION
(1)
(2)
(3)
10
10
10

FIG. 4

FIG. 5

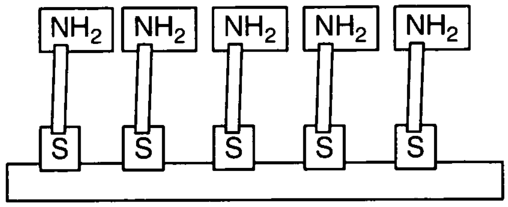


FIG. 6

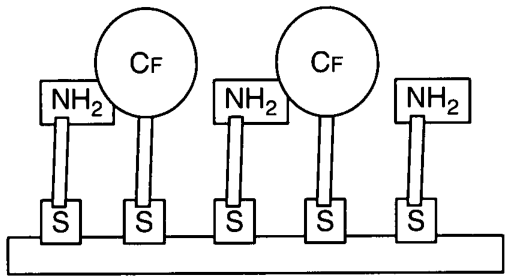


FIG. 7

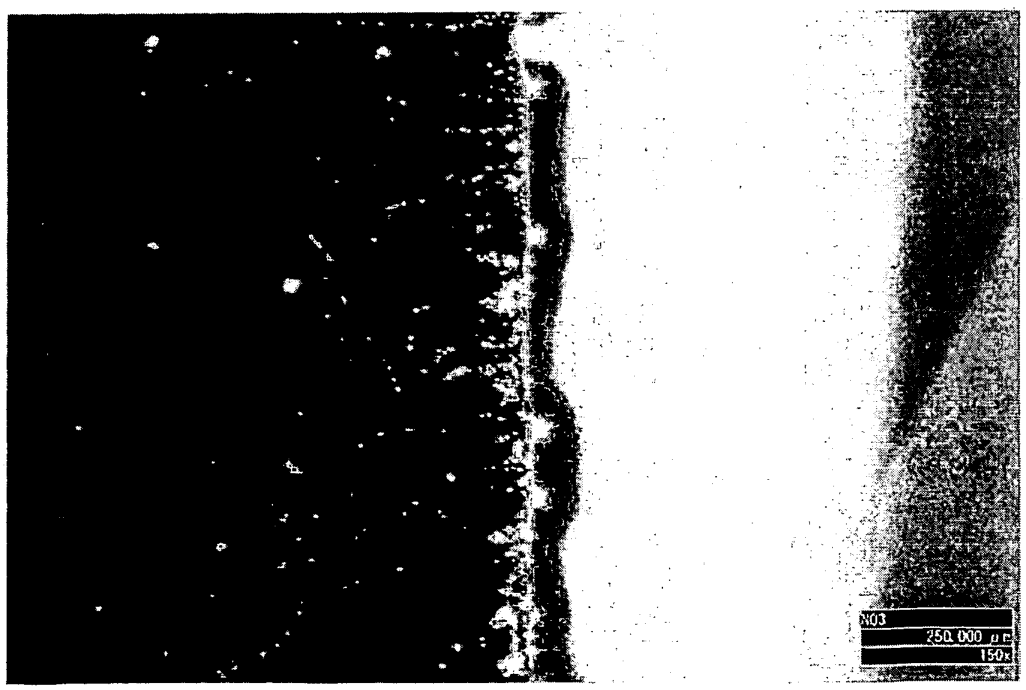

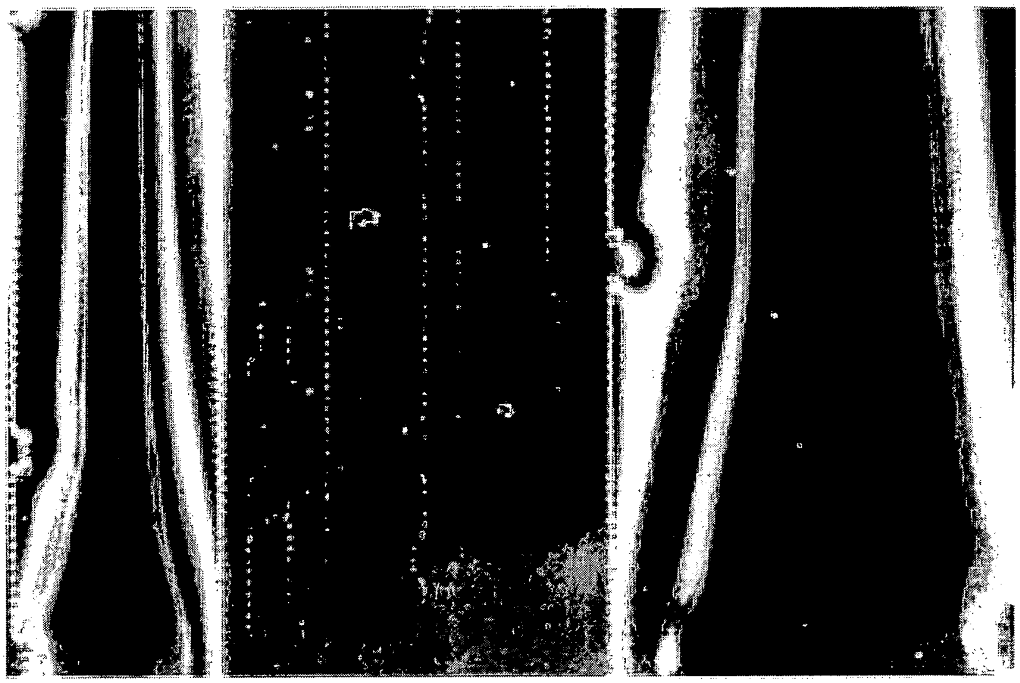

FIG. 8

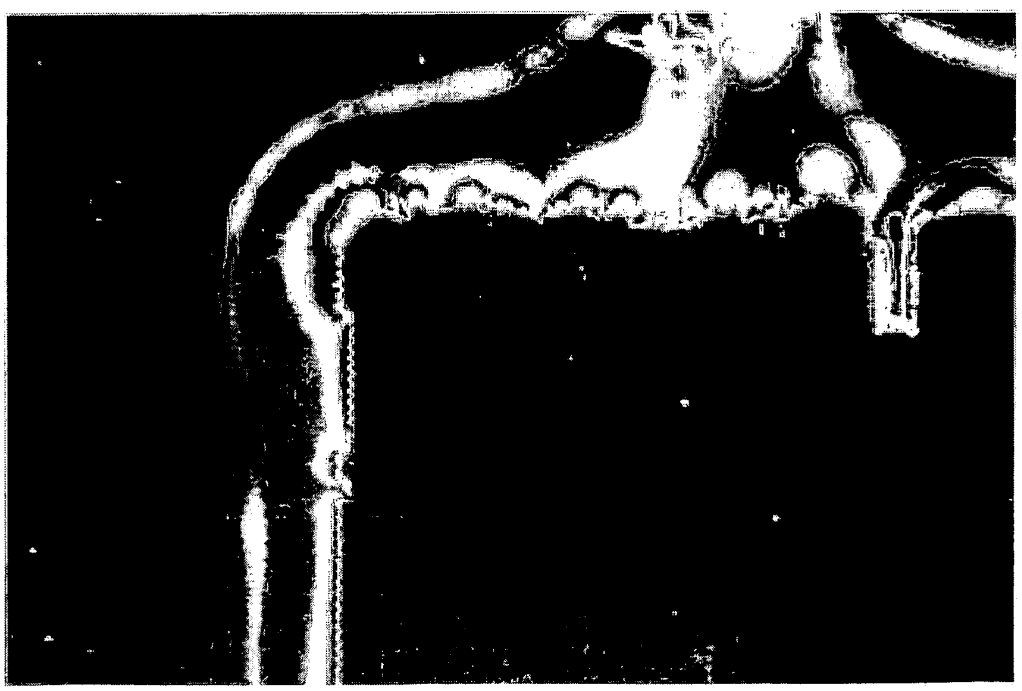

FIG. 9

FIG. 10

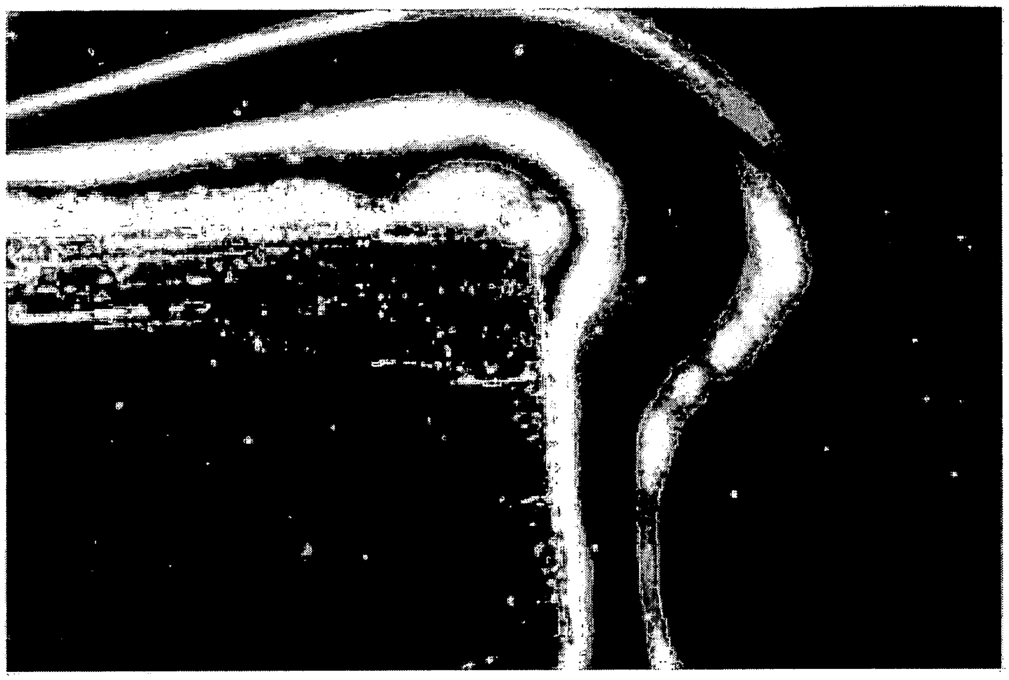

FIG. 11

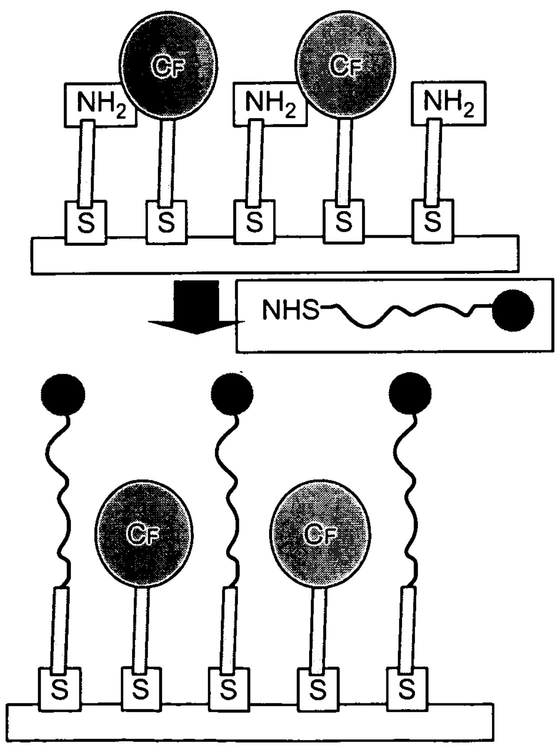

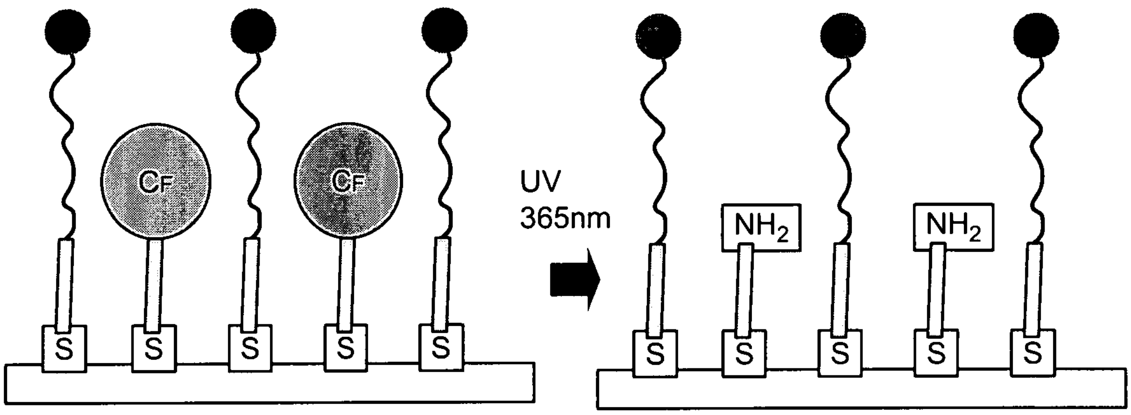


FIG. 12